# EUROPÄISCHE PATENTSCHRIFT

(11) **EP 0 854 140 B1**
(45) Veröffentlichungstag und Bekanntmachung des Hinweises auf die Patenterteilung: **06.11.2013**
(21) Anmeldenummer: 97121932.4
(22) Anmeldetag: 12.12.1997
(51) Int. Cl.: C07D 217/24, C07D 235/16, C07D 487/04, A61K 31/415, A61K 31/44, A61K 31/47, C07D 241/00, C07D 233/00, C07D 213/00, C07D 249/00

(54) **Vitronectinrezeptor-Antagonisten, deren Herstellung sowie deren Verwendung**
Vitronectin receptor antagonists, their production and their use
Récepteurs de la vitronectine, leur préparation et utilisation

(30) Priorität: 20.12.1996 DE 19653647
(43) Veröffentlichungstag der Anmeldung: 22.07.1998
(73) Patentinhaber: Sanofi-Aventis Deutschland GmbH, 65929 Frankfurt am Main (DE); Genentech, Inc., South San Francisco CA 94080-4990 (US)
(72) Erfinder: Wehner, Volkmar, Dr., 97657 Sandberg (DE); Stilz, Hans Ulrich, Dr., 65929 Frankfurt (DE); Peyman, Anuschirwan, Dr., 65779 Kelkheim (DE); Knolle, Jochen, Dr., 65830 Kriftel (DE); Ruxer, Jean-Marie, Dr., 92130 Issy les Moulineaux (FR); Carniato, Denis, Dr., 91460 Marcoussis (FR); Lefrancois, Jean-Michel, 931390 Livry Gargan (FR); Gadek, Thomas Richard, Dr., Oakland, CA 94611 (US); McDowell, Robert, Dr., San Francisco, CA 94114 (US)
(74) Vertreter: Santarelli

(56) Entgegenhaltungen:
- EP-A- 0 531 883
- EP-A- 0 741 133
- EP-A- 0 796 855
- WO-A-93/00095
- WO-A-94/14776
- WO-A-95/18111
- WO-A-95/32710
- WO-A-96/00574
- WO-A-96/00730
- WO-A-96/26190
- WO-A-97/01540
- WO-A-97/24119
- WO-A-97/24122
- WO-A-97/24124

## Beschreibung

Gegenstand der vorliegenden Erfindung sind Verbindungen der Formel I

A-B-D-E-F-G (I)

in der A, B, D, E, F und G die unten angegebenen Bedeutungen haben, sowie deren physiologisch verträgliche Salze und solche Verbindungen enthaltende pharmazeutische Zubereitungen, deren Herstellung und Verwendung als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen, wie z.B. Entzündungen, Krebs, Tumormetastasierung, cardiovaskuläre Erkrankungen wie Arteriosklerose oder Restenose, Retinopathien, Nephropathien, und Krankheiten, die auf einem unerwünschten Maß an Knochenresorption beruhen, wie z.B. Osteoporose.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption. Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", dem Bereich zwischen ihrer Zellmembran und der Knochenmatrix. Die saure Umgebung und die Proteasen bewirken den Abbau des Knochens.

Studien haben gezeigt, daß die Anlagerung von Osteoclasten an den Knochen durch Integrin-Rezeptoren auf der Zelloberfläche von Osteoclasten gesteuert wird.

Integrine sind eine Superfamilie von Rezeptoren, zu denen unter anderen der Fibrinogenrezeptor α_{IIb}β₃ auf den Blutplättchen und der Vitronectinrezeptor αᵥβ₃ gehören. Der Vitronectinrezeptor αᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor αᵥβ₃, der auf der Osteclastenmembran exprimiert wird, steuert den Prozeß der Anlagerung an den Knochen und der Knochenresorption und trägt somit zur Osteoporose bei. αᵥβ₃ bindet hierbei an Knochenmatrixproteine wie Osteopontin, Knochensialoprotein und Thrombospontin, die das Tripeptidmotiv Arg-Gly-Asp (oder RGD) enthalten.

Die erfindungsgemäßen Verbindungen der Formel I inhibieren als Vitronectinrezeptor-Antagonisten die Knochenresorption durch Osteoclasten. Knochenkrankheiten gegen die die erfindungsgemäßen Verbindungen eingesetzt werden können sind vor allem Osteoporose, Hypercalcämie, Osteopenie, z.B. hervorgerufen durch Metastasen, Zahnerkrankungen, Hyperparathyroidismus, periarticulare Erosionen bei rheumathoider Arthritis und Paget-Krankheit.
Ferner können die Verbindungen der Formel I zur Linderung, Vermeidung oder Therapie von Knochenerkrankungen, die durch eine Glucocortikoid-, Steroid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Horton und Mitarbeiter beschreiben RGD-Peptide und einen anti-Vitronectinrezeptor Antikörper (23C6), die den Zahnabbau durch Osteoclasten und das Wandern von Osteoclasten inhibieren (Horton et al.; Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteoclasten-Anheftung an den Knochen. Fischer et al. (Endocrinology, 1993, 132, 1411) konnten an der Ratte zeigen, daß Echistatin die Knochenresorption auch in vivo hemmt.

Der Vitronectinrezeptor αᵥβ₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Brooks et al. (Cell 1994, 79, 1157) zeigen, daß Antikörper gegen αᵥβ₃ oder αᵥβ₃-Antagonisten eine Schrumpfung von Tumoren bewirken können, indem sie die Apoptose von Blutgefäßzellen während der Angiogenese induzieren. Cheresh et al. (Science 1995, 270, 1500) beschreiben anti αᵥβ₃-Antikörper oder αᵥβ₃-Antagonisten, die bFGF induzierte Angiogeneseprozesse im Rattenauge inhibieren, was therapeutisch bei der Behandlung von Retinopathien nützlich sein könnte.

In der Patentanmeldung WO 94/12181 werden substituierte aromatische oder nichtaromatische Ringsysteme, in WO 94/08577 substituierte Heterocyclen als Fibrinogenrezeptor-Antagonisten und Inhibitoren der Blättchenaggregation beschrieben. Aus EP-A-0 528 586 und EP-A-0 528 587 sind Aminoalkyl- oder Heterocyclyl-substituierte Phenylalanin-Derivate, aus WO 95/32710 Arylderivate als Hemmstoffe der Knochenresorption durch Osteoclasten bekannt. Die WO 96/00574 und WO 96/26190 beschreiben Benzodiazepine als Vitronectin- bzw. Integrinrezeptor-Antagonisten. In der WO 96/00730 werden Fibrinogenrezeptorantagonisten-Template, insbesondere Benzodiazepine, die an einen Stickstoff tragenden 5-Ring geknüpft sind, als Vitronectinrezeptor-Antagonisten beschrieben. In den deutschen Patentanmeldungen P 19629816.4, P 19629817.2 und P 19610919.1 sowie der EP-A-0 796 855 werden substituierte aromatische Ringsysteme bzw. 5-Ringheterocyclen als Vitronectinrezeptor-Antagonisten beschrieben.

Gegenstand der vorliegenden Erfindung sind Verbindungen der Formel I,

A-B-D-E-F-G (I)

worin bedeuten:
- A =: A₁, mit
- A₁ =: R²R³N-C(=NR²)NR²C(O)-
- B: (C₁-C₄)-Alkandiyl,
- D: eine direkte Bindung, (C₁-C₄)-Alkandiyl,
oder c) 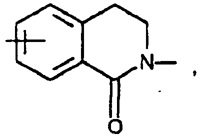 wobei V N ist und D^{a} CH₂-CH₂ ist; oder f) 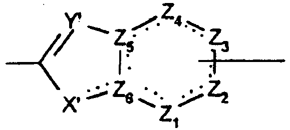 wobei bedeuten:
X' eine -NR^{2b} Grupp
wobei
R^{2b} ein Wasserstoff Atom ist
- Y',: ein Stickstoffatom,
- Z_{1'} Z₂, Z₃ und Z₄,: die gleich oder verschieden sein können, Methingruppen, Kohlenstoffatome, wobei einer der Reste Z₁ bis Z₄ ein Kohlenstoffatom enthalten muß und die drei anderen der Reste Z₁ bis Z₄ Methingruppen sind;
- Z₅ und Z₆: jeweils ein Kohlenstoffatom
- F: eine direkte Bindung, (C₁-C₆)-Alkandiyl, -CO-NR²-, -C≡C-, die jeweils ein- oder zweifach durch (C₁-C₄)-Alkyl substituiert sein können;
- G: 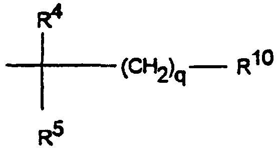
- R², R³: unabhängig voneinander H, (C₁-C₄)-Alkyl,
- R⁴: R¹⁶OC(O)NH-, R¹⁶C(O)NH-
- R⁵: H, (C₁-C₆)-Alkyl, (C₅-C₆)-Cycloalkyl, (C₅-C₆)-Cycloalkyl-(C₁-C₄)-alkyl, Trifluormethyl, Pentafluorethyl, Phenyl, Benzyl;
- R⁸: H, (C₁-C₄)-Alkyl, (C₅-C₆)-Cycloalkyl, (C₅-C₆)-Cycloalkyl-(C₁-C₂)-alkyl, Phenyl, Benzyl, Trifluormethyl, Pentafluorethyl;
- R¹⁰: C(O)R¹¹;
- R¹¹: OH, (C₁-C₆)-Alkoxy, Phenoxy, Benzyloxy, (C₁-C₄)-Alkylcarbonyloxy-(C₁-C₄)-alkoxy, NH₂, Mono- oder Di-(C₁-C₆-alkyl)-amino;

- R¹⁶: -(C₁₀-C₁₄)-Cycloalkyl, (C₁₀-C₁₄)-Cycloalkyl-(C₁-C₄)-alkyl, die gegebenenfalls 1 oder 2-fach durch (C₁-C₄)-Alkyl, Trifluormethyl, Phenyl, Benzyl, (C₁-C₄)-Alkoxy, Phenoxy, Benzyloxy, =O oder Mono- oder Di-((C₁-C₄)-alkyl)-amino substituiert sein können, wobei die Cycloalkylreste bevorzugt 1-Adamantyl oder 2-Adamantyl sind, die wie vorstehend beschrieben, substituiert sein können;
- n: 1 oder 2; und
- q: 0;
in allen ihren stereoisomeren Formen und Mischungen davon in allen Verhältnissen, und ihre physiologisch verträglichen Salze.

Unter einem Templat aus der Reihe der Fibrinogenrezeptorantagonisten versteht man den Mittelteil der Molekülstruktur (eines Fibrinogenrezeptorantagonisten), an den im Falle der Fibrinogenrezeptorantagonisten über Spacer eine basische und eine saure Gruppe angeknüpft sind, wobei die basische und/oder saure Gruppe gegebenenfalls in geschützter Form (Pro-Drug) vorliegen.

In den Fibrinogenrezeptorantagonisten ist die basische Gruppe im allgemeinen eine N-haltige Gruppe, wie z.B. Amidin oder Guanidin, die saure Gruppe im allgemeinen eine Carboxylfunktion, wobei die basische und saure Gruppe jeweils in geschützter Form vorliegen können.

Ein Fibrinogenrezeptorantagonist ist ein Wirkstoff, der die Bindung von Fibrinogen an den Blutplättchenrezeptor GPIIbIIIa inhibiert.

Ein Fibrinogenrezeptorantagonist besteht aus einem Mittelteil (Templat), an das über Spacer eine basische und eine saure Gruppe angeknüpft sind, wobei die basische und/oder saure Gruppe gegebenenfalls in geschützter Form (Pro-Drug) vorliegen.

Alkylreste können geradkettig oder verzweigt sein. Dies gilt auch, wenn sie Substituenten tragen oder als Substituenten anderer Reste auftreten, beispielsweise in Alkoxy-, Alkoxycarbonyl- oder Aralkylresten. Beispiele für geeignete (C₁-C₁₀)-Alkylreste sind: Methyl, Ethyl, Propyl, Butyl, Pentyl, Hexyl, Heptyl, Octyl, Decyl, Isopropyl, Isopentyl, Neopentyl, Isohexyl, 3-Methylpentyl, 2,3,5-Trimethylhexyl, sec-Butyl, tert-Pentyl. Bevorzugte Alkylreste sind Methyl, Ethyl, Propyl, Isopropyl, Butyl, Isobutyl, sec-Butyl und tert-Butyl.

Auch Alkenyl- und Alkinylreste können geradkettig und verzweigt sein. Beispiele für Alkenylreste sind Vinyl, 1-Propenyl, Allyl, Butenyl, 3-Methyl-2-butenyl, für Alkinylreste Ethinyl, 1-Propinyl oder Propargyl.

Cycloalkylreste können monocyclisch oder polycyclisch, z.B. bicyclisch oder tricyclisch, sein. Monocyclische Cycloalkylreste sind beispielsweise Cyclopropyl, Cyclobutyl, Cyclopentyl, Cyclohexyl, Cycloheptyl, Cyclooctyl, Cyclododecyl die aber auch durch beispielsweise (C₁-C₄)-Alkyl substituiert sein können. Als Beispiele für substituierte Cycloalkylreste seien 4-Methylcyclohexyl und 2,3-Dimethylcyclopentyl genannt.

Beispiele für Grundkörper monocyclischer (C₁₀-C₁₄)-Cycloalkylreste in R⁴, R⁵, R⁶,

R⁷ sind beispielsweise Cyclodecan und Cyclododecan.

Bicyclische und tricyclische Cycloalkylreste können unsubstituiert sein oder in beliebigen geeigneten Positionen durch eine oder mehrere Oxogruppen und/oder eine oder mehrere gleiche oder verschiedene (C₁-C₄)Alkylgruppen, z.B. Methyl- oder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Ein Beispiel für ein bicyclisches Ringsystem ist Dekalin (Decahydronaphthalin), für ein mit einer Oxogruppe substituiertes System 2-Decanon.

Beispiele für Grundkörper bicyclischer Ringsysteme sind das Norboman (= Bicyclo[2.2.1]heptan), das Bicyclo[2.2.2]octan und das Bicyclo[3.2.1]octan. Ein Beispiel für ein mit einer Oxogruppe substituiertes System ist der Campher (= 1,7,7-trimethyl-2-oxobicyclo[2.2.1]heptan).

Beispiele für Grundkörper tricyclischer Systeme sind das Twistan (= Tricyclo[4.4.0.0^{3,8}]decan, das Adamantan (= Tricyclo[3.3.1.1^{3,7}]decan), das Noradamantan (= Tricyclo[3.3.1.0^{3,7}]-nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Beispiele für Grundkörper tricyclischer (C₁₀-C₁₄)-Cycloalkylreste in R^{4,} R⁵, R⁶, R⁷ sind das Twistan (= Tricyclo[4.4.0.0.^{3,8}]decan, das Adamantan (= Tricyclo[3.3.1.1.^{3,7}]-nonan), das Tricyclo[5.3.2.0^{4,9}]dodecan, das Tricyclo[5.4.0.0^{2,9}]undecan oder das Tricyclo[5.5.1.0^{3,11}]tridecan.

Halogen bedeutet Fluor, Chlor, Brom, lod.

Beispiele für 6-gliedrige aromatische Ringsysteme sind Phenyl, Pyridyl, Pyridazinyl, Pyrimidinyl, Pyrazinyl, 1,3,5-Triazinyl, 1,2,4-Triazinyl, 1,2,3-Triazinyl, Tetrazinyl.

Aryl ist beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, wie Fluor, Chlor und Brom, Nitro, Amino, Trifluormethyl, Hydroxy, Methylendioxy, -OCH₂CH₂O-, -OC(CH₃)₂O-, Cyano, Hydroxycarbonyl, Aminocarbonyl, (C₁-C₄)-Alkoxycarbonyl, Phenyl, Phenoxy, Benzyl, Benzyloxy, (R¹⁷O)₂P(O), (R¹⁷O)₂P(O)-O- oder Tetrazolyl substituiert sein, wobei R¹⁷ H, (C₁-C₁₀)-Alkyl, (C₆-C₁₄)-Aryl oder (C₆-C₁₄)-Aryl-(C₁-C₈)-alkyl bedeutet.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Bevorzugt sind in zweifach substituierten Phenylresten die beiden Substituenten in der 3- und der 4-Position, bezogen auf die Verknüpfungsstelle, angeordnet.

Arylgruppen können ferner mono- oder polycyclische aromatische Ringsysteme darstellen, worin 1 bis 5 C-Atome durch 1 bis 5 Heteroatome ersetzt sein können, wie z.B. 2-Pyridyl, 3-Pyridyl, 4-Pyridyl, Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, ß-Carbolinyl, oder ein benz-anelliertes, cyclopenta-, cyclohexa- oder cyclohepta-anelliertes Derivat dieser Reste. Diese Heterocyclen können mit den gleichen Substituenten wie die vorstehend genannten carbocyclischen Arylsysteme substituiert sein.

In der Reihe dieser Arylgruppen sind bevorzugt mono- oder bicyclische aromatische Ringsysteme mit 1 bis 3 Heteroatome aus der Reihe N, O, S, die mit 1 bis 3 Substituenten aus der Reihe (C₁-C₆)-Alkyl, (C₁-C₆)-Alkoxy, F, Cl, NO₂, NH₂, CF₃, OH, (C₁-C₄)-Alkoxycarbonyl, Phenyl, Phenoxy, Benzyloxy oder Benzyl substituiert sein können.

Besonders bevorzugt sind hierbei mono- oder bicyclische aromatische 5- bis 10-Ringsysteme mit 1 bis 3 Heteroatomen aus der Reihe N, O, S, die mit 1 bis 2 Substituenten aus der Reihe (C₁-C₄)-Alkyl, (C₁-C₄)-Alkoxy, Phenyl, Phenoxy, Benzyl oder Benzyloxy substituiert sein können.

L- oder D-Aminosäuren können natürliche oder unnatürliche Aminosäuren sein. Bevorzugt sind α-Aminosäuren. Beispielsweise seien genannt (vgl. Houben-Weyl, Methoden der organischen Chemie, Band XV/1 und 2, Georg Thieme Verlag, Stuttgart, 1974):

Aad, Abu, γAbu, ABz, 2ABz, εAca, Ach, Acp, Adpd, Ahb, Aib, βAib, Ala, ßAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, ßLys, ΔLys, Met, Mim, Min, nArg, Nle, Nva, Oly, Om, Pan, Pec, Pen, Phe, Phg, Pic, Pro, ΔPro, Pse, Pya, Pyr, Pza, Qin, Ros, Sar, Sec, Sem, Ser, Thi, ßThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure;
ferner:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]-heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Decahydrocycloocta[c]pyrrol-2-carbonsäure;
Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

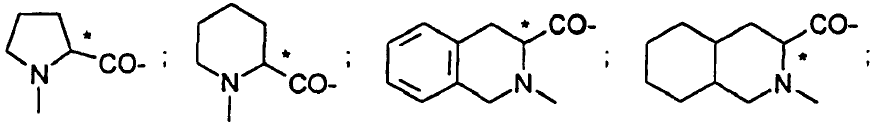

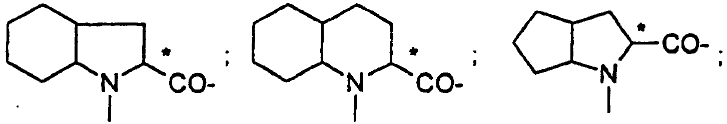

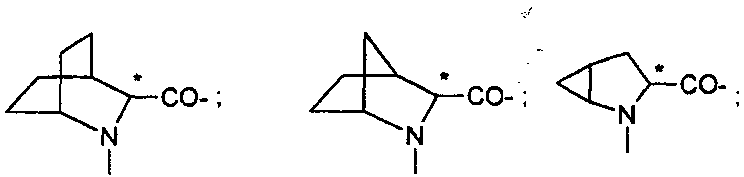

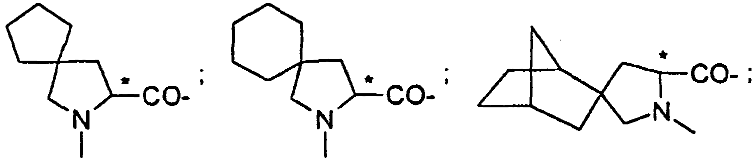

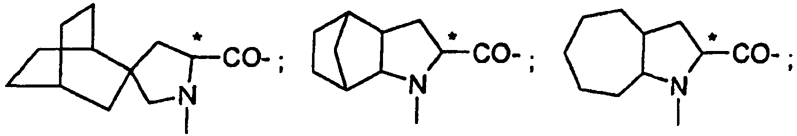

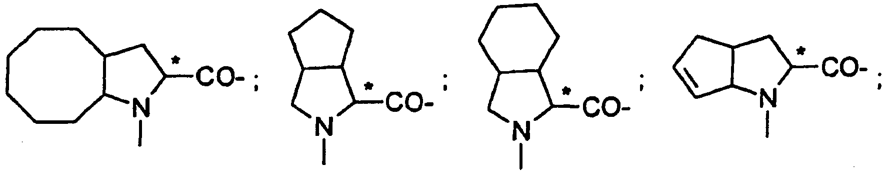

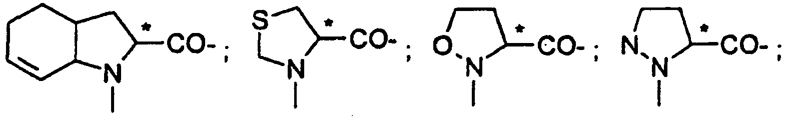

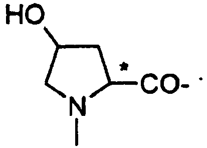

Die oben genannten Resten zugrundeliegenden Heterocyclen sind beispielsweise bekannt aus US-A-4,344,949; US-A 4,374,847;
US-A 4,350,704; EP-A 29,488; EP-A 31,741; EP-A 46,953; EP-A 49,605;
EP-A 49,658; EP-A 50,800; EP-A 51,020; EP-A 52,870; EP-A 79,022;
EP-A 84,164; EP-A 89,637; EP-A 90,341; EP-A 90,362; EP-A 105,102;
EP-A 109,020; EP-A 111, 873; EP-A 271,865 und EP-A 344, 682.

Ferner können die Aminosäuren auch als Ester bzw. Amide vorliegen, wie z.B. Methylester, Ethylester, Isopropylester, Isobutylester, tert-Butylester, Benzylester, unsubstituiertes Amid, Ethylamid, Semicarbazid oder ω-Amino-(C₂-C₈)-alkylamid.

Funktionelle Gruppen der Aminosäuren können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Solche Salze werden beispielsweise von Verbindungen der Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin, Ethanolamin oder Tris-(2-hydroxy-ethyl)-amin. Verbindungen der Formel I, welche basische Gruppen, z.B. eine Aminogruppe, eine Amidinogruppe oder eine Guanidinogruppe enthalten, bilden mit anorganischen Säuren, wie z.B. Salzsäure, Schwefelsäure oder Phosphorsäure, und mit organischen Carbon- oder Sulfonsäuren, wie z.B. Essigsäure, Citronensäure, Benzoesäure, Maleinsäure, Fumarsäure, Weinsäure, Milchsäure, Methansulfonsäure oder p-Toluolsulfonsäure Salze.

Die erfindungsgemäßen Verbindungen der Formel I können optisch aktive Kohlenstoffatome, die unabhängig voneinander R- oder S-Konfigurationen haben können, enthalten und können somit in Form reiner Enantiomerer oder reiner Diastereomerer oder in Form von Enantiomerengemischen oder Diastereomerengemischen vorliegen. Sowohl reine Enantiomere und Enantiomerengemische in allen Verhältnissen als auch Diastereomere und Diastereomerengemische in allen Verhältnissen sind Gegenstand der vorliegenden Erfindung.

Die erfindungsgemäßen Verbindungen der Formel I können, unabhängig voneinander als E/Z-Isomerengemische vorliegen. Gegenstand der vorliegenden Erfindung sind sowohl reine E- bzw. Z-Isomere als auch E/Z-Isomerengemische.

Diastereomere einschließlich E/Z-Isomere können durch Chromatographie in die Einzelisomeren aufgetrennt werden. Racemate können entweder durch Chromatographie an chiralen Phasen oder durch Racematspaltung in die beiden Enantiomere aufgetrennt werden.

Die erfindungsgemäßen Verbindungen der Formel **I** können darüber hinaus bewegliche Wasserstoffatome enthalten, also in verschiedenen tautomeren Formen vorliegen. Auch alle diese Tautomeren sind Gegenstand der vorliegenden Erfindung.

Bevorzugt sind Verbindungen der Formel I, die selektive Vitronectinrezeptor-Antagonisten, insbesondere in Bezug zum Fibrinogenrezeptor, darstellen, d.h. die stärkere Inhibitoren des Vitronectinrezeptors als des Fibrinogenrezeptors sind.

Bevorzugt sind insbesondere Verbindungen der Formel I, die selektive Vitronectinrezeptoren-Antagonisten darstellen und in denen der Abstand zwischen R¹⁰ und dem ersten N-Atom in A₁ 12 bis 13, in A₂ 11 bis 12 kovalente Bindungen entlang des kürzesten Weges zwischen diesen Atomen beträgt, wie nachfolgend exemplarisch für

A₁ = 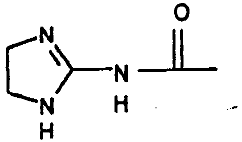 und R¹⁰ = COOH dargestellt:

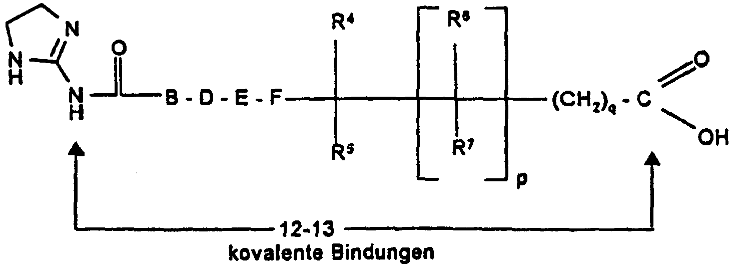

Bevorzugt sind ferner Verbindungen der Formel I, in denen mindestens ein Rest aus der Reihe R⁴, R⁵, R⁶, R⁷ einen lipophilen Rest darstellt.

Beispiele für lipophile Reste in der Reihe R⁴, R⁵, R⁶, R⁷ sind z.B. Neopentyl, Cyclohexyl, Adamantyl, Cyclohexyl-(C₁-C₈)-alkyl, Adamantyl-(C₁-C₈)-alkyl, Phenyl, Naphthyl, Phenyl-(C₁-C₈)-alkyl, Naphthyl-(C₁-C₈)-alkyl, Cyclohexylmethylcarbonylamino, 1-Adamantylmethyloxycarbonylamino oder Benzyloxycarbonylamino bzw. allgemein Reste, in denen R⁸ z.B. Neopentyl, Cyclohexyl, Adamantyl, Cyclohexyl-(C₁-C₈)-alkyl, Adamantyl-(C₁-C₈)-alkyl, Phenyl, Naphthyl, Phenyl-(C₁-C₈)-alkyl, Naphthyl-(C₁-C₈)-alkyl bedeutet.

Ein weiterer Gegenstand der vorliegenden Erfindung besteht darin, daß man einen an sich bekannten Fibrinogenrezeptorantagonisten in einen selektiven Vitronectinrezeptorantagonisten überführen kann, indem man die basische Gruppe (mit Spacer) eines Fibrinogenrezeptorantagonisten durch A-B-D ersetzt, der wie in Formel I definiert ist, wobei der Abstand zwischen R¹⁰ und dem ersten N-Atom in A₁ 12 bis 13, kovalente Bindungen entlang des kürzesten Weges zwischen diesen Atomen beträgt.

Verbindungen der Formel I können generell, beispielsweise im Zuge einer konvergenten Synthese, durch Verknüpfung zweier oder mehrerer Fragmente, die sich retrosynthetisch aus der Formel I ableiten lassen, hergestellt werden. Bei der Herstellung der Verbindungen der Formel I kann es generell im Laufe der Synthese nötig sein, funktionelle Gruppen, die im jeweiligen Syntheseschritt zu unerwünschten Reaktionen oder Nebenreaktionen führen könnten, durch eine dem Syntheseproblem angepaßte Schutzgruppenstrategie temporär zu blockieren, was dem Fachmann bekannt ist. Die Methode der Fragmentverknüpfung ist nicht auf die nachfolgenden Beispiele beschränkt, sondern allgemein für Synthesen der Verbindungen der Formel I anwendbar.

Beispielsweise können Verbindungen der Formel I des Typs

A-B-D-E-C(O)NR²-G,

mit F = C(O)NR² durch Kondensation einer Verbindung der Formel II

A-B-D-E-M II,

wobei M für Hydroxycarbonyl, (C₁-C₆)-Alkoxycarbonyl, aktivierte Carbonsäurederivate wie Säurechloride, Aktivester oder gemischte Anhydride steht, mit HNR²-G hergestellt werden.

Zur Kondensation zweier Fragmente unter Bildung einer Amidbindung verwendet man vorteilhafterweise die an sich bekannten Kupplungsmethoden der Peptidchemie (siehe z.B. Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert-Butylester eingesetzt werden. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird.

Verbindungen der Formel I mit R¹⁰ = SO₂R¹¹ werden beispielsweise hergestellt, in dem man Verbindungen der Formel I mit R¹⁰ = SH nach literaturbekannten Verfahren (vgl. Houben-Weyl, Methoden der Organischen Chemie, Bd. E12/2, Georg Thieme Verlag, Stuttgart 1985, S. 1058ff) zu Verbindungen der Formel I mit R¹⁰ = SO₃H oxidiert, aus denen dann direkt oder über entsprechende Sulfonsäurehalogenide durch Veresterung oder Knüpfung einer Amidbindung die Verbindungen der Formel I mit R¹⁰ = SO₂R¹¹ (R¹¹ ≠ OH) hergestellt werden. Oxidationsempfindliche Gruppen im Molekül, wie z.B. Amino-, Amidino- oder Guanidinogruppen werden, falls erforderlich, vor Durchführung der Oxidation durch geeignete Schutzgruppen geschützt.

Verbindungen der Formel I mit R¹⁰ = S(O)R¹¹ werden beispielsweise hergestellt, in dem man Verbindungen der Formel I mit R¹⁰ = SH in das entsprechende Sulfid (R¹⁰ = S^{⊖}) überführt und anschließend mit meta-Chlorperbenzoesäure zu den Sulfinsäuren (R¹⁰ = SO₂H) oxidiert (vgl. Houben-Weyl, Methoden der Organischen Chemie, Bd. E11/1, Georg Thieme Verlag, Stuttgart 1985, S. 618f), aus denen nach literaturbekannten Methoden die entsprechenden Sulfinsäureester oder -amide R¹⁰ = S(O)R¹¹ (R¹¹ ≠ OH) hergestellt werden können. Generell können auch andere literaturbekannte Methoden zur Herstellung von Verbindungen der Formel I mit R¹⁰ = S(O)ₙR¹¹ (n = 1, 2) Anwendung finden (vgl. Houben-Weyl, Methoden der Organischen Chemie, Bd. E11/1, Georg Thieme Verlag, Stuttgart 1985, S. 618ff oder Bd. E11/2, Stuttgart 1985, S. 1055ff).

Verbindungen der Formel I mit R¹⁰ = P(O)(R¹¹)ₙ (n = 1, 2) werden nach literaturbekannten Verfahren (vgl. Houben-Weyl, Methoden der Organischen Chemie, Bd. E1 und E2, Georg Thieme Verlag, Stuttgart 1982) aus geeigneten Vorstufen aufgebaut, wobei die gewählte Synthesemethode dem Zielmolekül anzupassen ist.

Verbindungen der Formel I mit R¹⁰ = C(S)R¹¹ können nach Literaturverfahren hergestellt werden (vgl. Houben-Weyl, Methoden der Organischen Chemie, Bd. E5/1 und E5/2, Georg Thieme Verlag, Stuttgart 1985).

Verbindungen der Formel I mit R¹⁰ = S(O)ₙR¹¹ (n = 1, 2), P(O)(R¹¹)ₙ (n = 1, 2) oder C(S)R¹¹ können natürlich auch durch Fragmentverknüpfung, wie vorstehend beschrieben, hergestellt werden, was beispielsweise ratsam ist, wenn in F-G der Formel I z.B. eine (käufliche) Aminosulfonsäure, Aminosulfinsäure, Aminophosphonsäure oder Aminophosphinsäure oder daraus abgeleitete Derivate, wie Ester oder Amide, enthalten sind.

Verbindungen der Formel I in denen A = A₁ =
R²R³N-C(=NR²)-NR²-C(O)- oder cyclische Acylguanidine des Typs 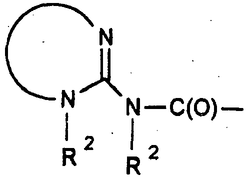 bedeutet, können beispielsweise hergestellt werden, indem man eine Verbindung der Formel III,

Q(O)C-B-D-E-F-G III

in der Q für eine leicht nukleophil substituierbare Abgangsgruppe steht, mit dem entsprechenden Guanidin(derivat) des Typs 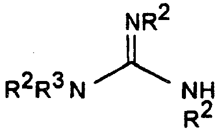 oder dem cyclischen Guanidin(derivat) des Typs 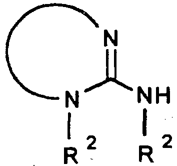 umsetzt.

Die aktivierten Säurederivate der Formel III, worin Q eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäuren (Q = OH) oder Carbonsäurechloriden (Q = CI). Letztere erhält man wiederum in an sich bekannter Weise aus den zugrundeliegenden Carbonsäuren (Q = OH) beispielsweise durch Reaktion mit Thionylchlorid.

Neben den Carbonsäurechloriden (Q = CI) lassen sich auch weitere aktivierte Säurederivate des Typs Q(O)C- in an sich bekannter Weise direkt aus den zugrundeliegenden Carbonsäuren (Q = OH) herstellen, wie beispielsweise die Methylester (Q = OCH₃) durch Behandeln mit gasförmigem HCl in Methanol, die Imidazolide (Q = 1-Imidazolyl) durch Behandeln mit Carbonyldiimidazol [vgl. Staab, Angew. Chem. Int. Ed. Engl. 1, 351-367 (1962)], die gemischten Anhydride (Q = C₂H₅OC(O)O bzw. TosO) mit Cl-COOC₂H₅ bzw. Tosylchlorid in Gegenwart von Triethylamin in einem inerten Lösungsmittel. Die Aktivierung der Carbonsäuren kann auch mit Dicyclohexylcarbodiimid (DCCI) oder mit O-[(Cyano(ethoxycarbonyl)methylen)amino]-1,1,3,3-tetramethyluronium-tetrafluorborat ("TOTU") [Weiss und Krommer, Chemiker-Zeitung 98, 817 (1974)] und anderer in der Peptidchemie gebräuchlichen Aktivierungs-Reagentien erfolgen. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel III mit dem jeweiligen Guanidin(derivat) erfolgt in an sich bekannter Weise in einem protischen oder aprotischen polaren aber inerten organischen Lösungsmittel. Dabei haben sich bei der Umsetzung der Methylester (Q = OMe) mit den jeweiligen Guanidinen Methanol, Isopropanol oder Tetrahydrofuran (THF) bei Temperaturen von 20°C bis zur Siedetemperatur dieser Lösungsmittel bewährt. Bei den meisten Umsetzungen von Verbindungen der Formel III mit salzfreien Guanidinen wird vorteilhaft in aprotischen inerten Lösungsmitteln wie THF, Dimethoxyethan, Dioxan gearbeitet. Aber auch Wasser kann unter Gebrauch einer Base (wie beispielsweise NaOH) als Lösungsmittel bei der Umsetzung von Verbindungen der Formel III mit Guanidinen verwendet werden. Wenn Q = CI bedeutet, arbeitet man vorteilhaft unter Zusatz eines Säurefängers, z.B. in Form von überschüssigem Guanidin(derivat) zur Abbindung der Halogenwasserstoffsäure.

Verbindungen der Formel I, in denen A = A₁ = R¹R²N-C(=NR²)-NR²-C(S)- oder 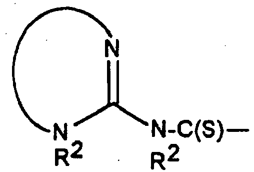 bedeutet, können wie bei der Synthese von entsprechenden offenkettigen oder cyclischen Acylguanidin(derivaten) beschrieben hergestellt werden, indem man eine Verbindung der Formel XI

Q(S)C-B-D-E-F-G (XI)

in der Q wie vorstehend definiert ist, mit dem entsprechenden Guanidin(derivat) des Typs 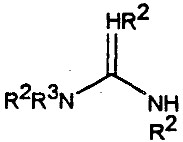 bzw. dem cyclischen Guanidin(derivat) des Typs 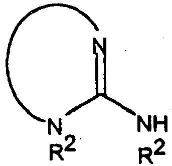 wie vorstehend beschrieben, umsetzt.

Verbindungen der Formel I, in denen A = A₁ = ein Sulfonyl- oder Sulfoxylguanidin des Typs R²R³N-C(=NR²)-NR²-S(O)ₙ- (n = 1, 2) bzw. 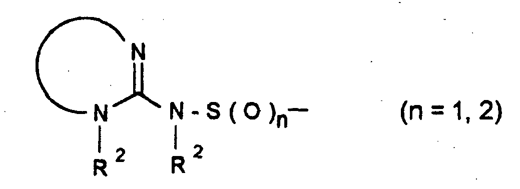 darstellt, werden nach literaturbekannten Verfahren durch Reaktion von R²R³N-C(=NR³)NR²H bzw. 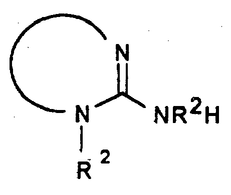 mit Sulfin- oder Sulfonsäurederivaten der Formel IV

Q-S(O)ₙ-B-D-E-F-G IV

in denen Q z.B. gleich Cl oder NH₂ bedeutet, analog S. Birtwell et al., J. Chem. Soc. (1946) 491 oder Houben Weyl, Methoden der Organischen Chemie, Bd. E4, Georg Thieme Verlag, Stuttgart 1983; S. 620 ff, hergestellt.

Verbindungen der Formel I, in denen F für -R²N-C(O)-NR²- oder -R²N-C(S)-NR²-steht, werden beispielsweise hergestellt, indem man eine Verbindung der Formel VII

A-B-D-E-NHR² VII

mit einem Isocyanat OCN-G oder Isothiocyanat SCN-G nach literaturbekannten Verfahren umsetzt.

Verbindungen der Formel I, in denen F für -C(O)NR²-, -SO₂NR²- oder -C(O)O-steht, können z. B. durch Umsetzung von

A-B-D-E-C(O)Q bzw. A-B-D-E-SO₂Q

(Q ist eine leicht nukleophil substituierbare Abgangsgruppe, wie z.B. OH, Cl, OMe etc.) mit HR²N-G bzw. HO-G nach Literaturverfahren erhalten werden.

Verbindungen der Formel I, in denen A = A₂ = 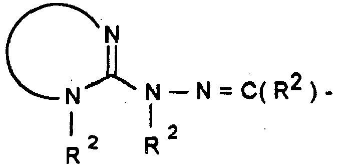 bedeutet, werden beispielsweise durch Kondensation von 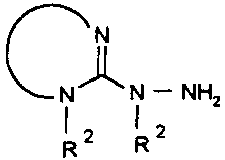 mit Ketonen oder Aldehyden des Typs O=C(R²)- oder entsprechenden Acetalen oder Ketalen nach gängigen Literaturverfahren, beispielsweise analog N. Desideri et al., Arch. Pharm. 325 (1992) 773-777, A. Alves et al., Eur. J. Med. Chem. Chim. Ther. 21 (1986) 297-304, D. Heber et al., Pharmazie 50 (1995) 663-667, T.P. Wunz et al., J. Med. Chem. 30 (1987) 1313-1321, K.-H. Buchheit et al., J. Med. Chem. 38 (1995), 2331-2338, hergestellt.

Obige Guanylhydrazone können gegebenenfalls als E/Z-Isomerengemische anfallen, die nach gängigen Chromatographieverfahren getrennt werden können.

Verbindungen der Formel I, in denen D -C≡C- ist, können beispielsweise hergestellt werden, indem man eine Verbindung der Formel IX

X-E-F-G IX

in der X = I oder Br bedeutet, mit einer Verbindung des Typs A-B-C≡CH in einer Palladium-katalysierten Reaktion, wie z.B. bei A. Arcadi et al., Tetrahedron Lett. 1993, 34, 2813 oder E.C. Taylor et al., J. Org. Chem. 1990, 55, 3222 beschrieben, umsetzt.

Analog können Verbindungen der Formel I, in denen F gleich -C≡C- ist beispielsweise durch Verknüpfung von Verbindungen der Formel X

A-B-D-E-X X

in der X I oder Br bedeutet, mit einer Verbindung des Typs HC≡C-G in einer Palladium-katalysierten Reaktion hergestellt werden.

Die Synthese der Fibrinogenrezeptorantagonisten-Template E erfolgt wie in den jeweiligen Patenten, Patentanmeldungen oder Publikationen beschrieben, wobei während der Templatsynthese, oder anschließend, vorzugsweise bereits während der Templatsynthese, funktionelle Gruppen in das Templat eingebaut oder an das Templat geknüpft werden, die die spätere Anknüpfung von A-B-D und F-G durch Fragmentverknüpfung erlauben, wie nachfolgend für ein Templat aus WO 94/18981 exemplarisch beschrieben:

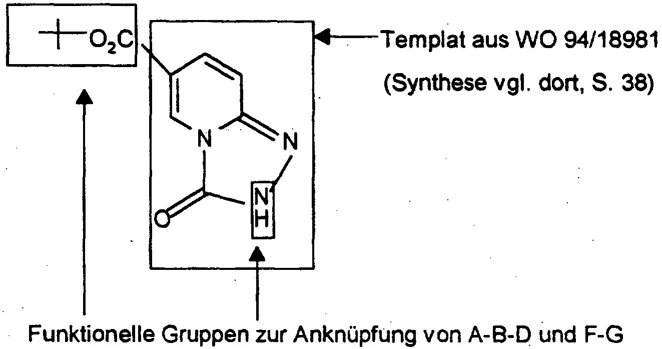

Beispiel für die Anknüpfung von A-B-D und F-G

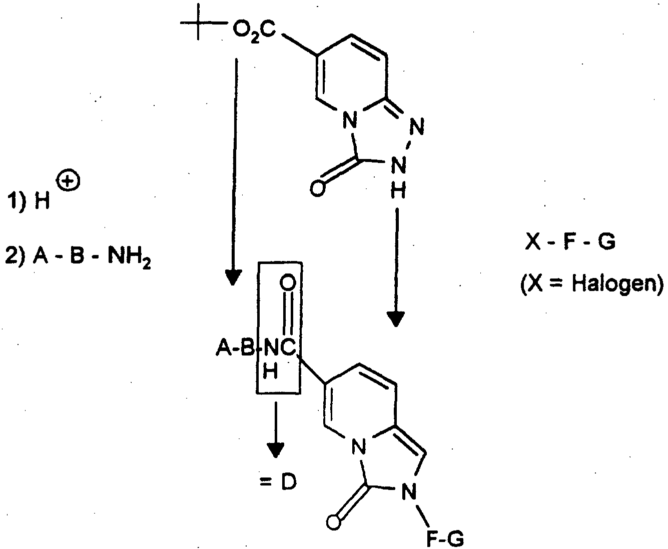

Literaturbekannte Herstellungsverfahren sind z.B. in J. March, Advanced Organic Chemistry, Third Edition (John Wiley & Sons, 1985) beschrieben.

Die Verbindungen der Formel I und ihre physiologisch verträglichen Salze können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben üblichen pharmazeutisch einwandfreien Träger- und Zusatzstoffen enthalten. Die Zubereitungen enthalten normalerweise etwa 0.5 bis 90 Gew.-% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, z.B. in Form von Suppositorien, oder parenteral, z.B. in Form von Injektions- oder Infusionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder deren physiologisch verträglichen Salze enthalten; ferner neben mindestens einer Verbindung der Formel I noch einen oder mehrere andere therapeutisch wirksame Stoffe.

Die Dosis kann innerhalb weiter Grenzen variieren und ist in jedem einzelnen Fall den individuellen Gegebenheiten anzupassen.

Bei oraler Verabreichung kann die Tagesdosis zwischen 0.01 bis 100 mg/kg, vorzugsweise 0.1 bis 5 mg/kg, insbesondere 0.3 bis 0.5 mg/kg Körpergewicht zur Erzielung wirksamer Ergebnisse, betragen. Auch bei intravenöser Applikation beträgt die Tagesdosis im allgemeinen etwa 0.01 bis 100 mg/kg, vorzugsweise 0.05 bis 10 mg/kg Körpergewicht. Die Tagesdosis kann, insbesondere bei der Applikation größerer Mengen, in mehrere, z.B. 2, 3 oder 4 Teilverabreichungen aufgeteilt werden. Gegebenenfalls kann es, je nach individuellem Verhalten, erforderlich werden, von der angegebenen Tagesdosis nach oben oder nach unten abzuweichen.

Außer als Arzneimittelwirkstoffe können die Verbindungen der Formel I in Diagnoseverfahren, zum Beispiel in in vitro-Diagnosen, oder als Hilfsmittel bei biochemischen Untersuchungen eingesetzt werden, bei denen eine Hemmung des Vitronectinrezeptors beabsichtigt ist.

### Beispiele

Die Produkte wurden über Massenspektren und/oder NMR-Spektren identifiziert.

### Beispiel 1

### (2S)-3-[5-(2-Guanidinocarbonyl-ethyl)-4,5,6,7-tetrahydro-4-oxo-pyrazolo[1,5-a]-pyrazin-2-carbonylamino]-2-[benzyloxycarbonylamino]-propionsäure (1.8)

Die Synthese erfolgte nach folgender Reaktionssequenz:

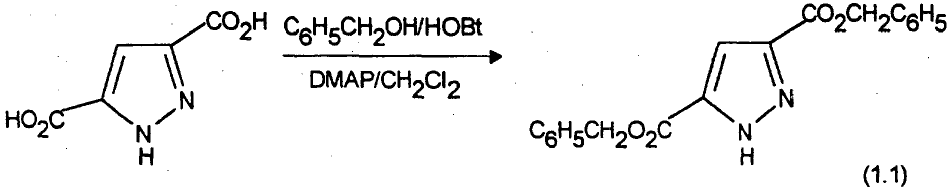

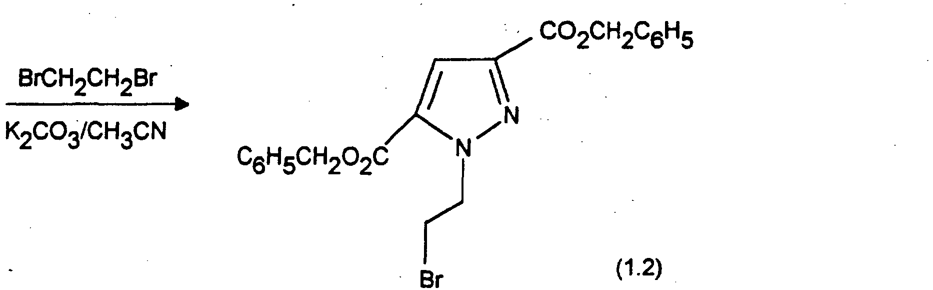

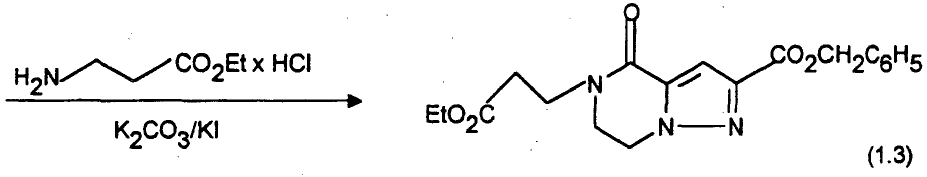

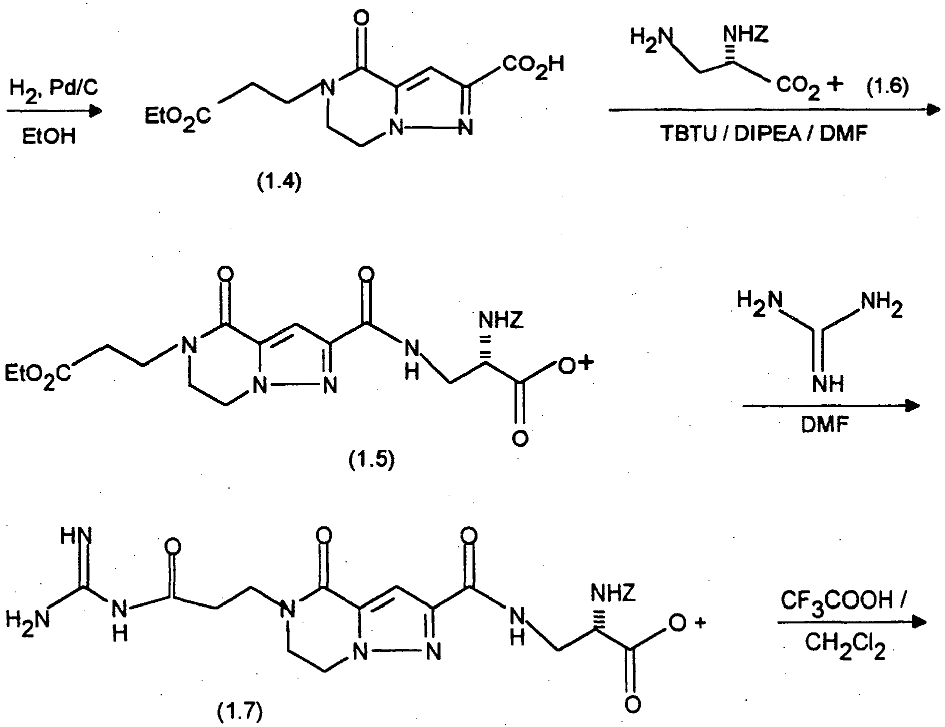

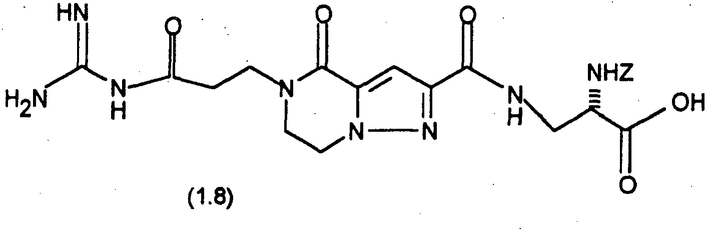

### Pyrazol-3,5-dicarbonsäure-dibenzylester (1.1)

Zu einer Mischung von 5.0 g (28.7 mmol) Pyrazol-3,5-dicarbonsäuremonohydrat in 150 ml Methylenchlorid gab man 0.77 g (6.3 mmol) 4-Dimethylaminopyridin, 8.53 g (63.1 mmol) 1-Hydroxybenzotriazolhydrat, 12.1 g (63.1 mmol) 1-(3-Dimethylaminopropyl)-3-ethylcarbodiimid-hydrochlorid und 6.5 ml (6.8 g, 62.8 mmol) Benzylalkohol. Man rührte unter Stickstoffatmosphäre etwa 20 Stunden bei Raumtemperatur, bis die Reaktion beendet war (Dünnschichtchromatogramm (DC): Kieselgel; Diethylether/Pentan 50/50). Das Reaktionsgemisch wurde mit 100 ml Wasser verdünnt, filtriert, die organische Phase abgetrennt und mit 2 x 100 ml Wasser, 2 x 50 ml 5 % Essigsäure und 100 ml Wasser gewaschen. Nach Trocknen über Natriumsulfat wurde das Lösungsmittel im Vakuum entfernt. Der Rückstand wurde über Kieselgel chromatographiert (Methanol/Methylenchlorid 5/95).
Ausbeute: 4.3 g (45 %)
Schmelzpunkt: 120°C (Koflerbank)
DC: R_{f} = 0.3 (Kieselgel; Diethylether/Pentan 50/50)
IR (CHCl₃): 3420 (=C-NH); 1728 (CO); 1558-1495 cm-1 (C=C).
¹H-NMR (CDCl₃) 250 MHz: 5.38 (s, 2CH₂); 7.38 (s, CH); 7.37 (m, Ph); 11.5 ppm (bs, NH).

| | | | | |
|---|---|---|---|---|
| CHN-Analyse: | Berechn. | C 67.85; | H 4.79; | N 8.33 |
| | Gefund. | C 67.5; | H 4.6; | N 8.2 |

### 1-(2-Bromo-ethyl)-pyrazol-3,5-dicarbonsäure-dibenzylester (1.2)

400 mg (1.19 mmol) Pyrazol-3,5-dicarbonsäure-dibenzylester (1.1), 225 mg (1.63 mmol) Kaliumcarbonat und 1.04 ml (2.27 g, 12.0 mmol) 1,2-Dibromethan in 10 ml Acetonitril wurden in einer Stickstoffatmosphäre unter Rückfluß erhitzt, bis die Reaktion beendet war (ca. 2 h; DC: Kieselgel; Methanol/Methylenchlorid 5/95). Das Reaktionsgemisch wurde filtriert und im Vakuum eingeengt. Der Rückstand wurde über Kieselgel chromatographiert (Methylenchlorid, dann Methanol/Methylenchlorid 2/98). Ausbeute: 450 mg (85 %).
Schmelzpunkt: 112°C (Koflerbank)
DC: R_{f} = 0.3 (Kieselgel; Methylenchlorid)

IR (CHCl₃): 1727 (CO); 1588-1529-1498 cm⁻¹ (C=C).

¹H-NMR (CDCl₃) 250 MHz: 3.72 (t, CH₂); 5.03 (t, CH₂); 5.34 (s, CH₂Ph); 5.38 (s, CH₂Ph); 7.30-7.47 ppm (m, 11CH).

| | | | | | |
|---|---|---|---|---|---|
| CHN-Analyse: | Berechn. | C 56 90; | H 4.32; | N 6.32; | Br 18.02 |
| | Gefund. | C 56.9; | H 4.2; | N 6.2; | Br 18.0 |

### 5-(2-Ethoxycarbonyl-ethyl)-4,5,6,7-tetrahydro-4-oxo-pyrazolo[1,5-a]pyrazin-2-carbonsäure-benzylester (1.3)

Man erhitzte unter Stickstoffatmosphäre eine Mischung von 4.00 g (9.02 mmol) 1-(2-Bromo-ethyl-pyrazol-3,5-dicarbonsäure-dibenzylester (1.2), 3.11 g (22.5 mmol) Kaliumcarbonat, 300 mg Kaliumiodid und 1.52 g (9.90 mmol) 3-Aminopropionsäureethylester-hydrochlorid in 250 ml Dioxan unter Rückfluß, bis die Reaktion beendet war (ca. 48 h; Dünnschicht; Kieselgel;
Methanol/Methylenchlorid 5/95). Das Reaktionsgemisch wurde filtriert und im Vakuum eingeengt. Der Rückstand wurde über Kieselgel chromatographiert (Methylenchlorid bis Methanol/Methylenchlorid 1/99). Ausbeute: 1.60 g (48 %), Öl.

DC: R_{f} = 0.4 (Kieselgel; Methanol/Methylenchlorid 5/95)

IR (CHCl₃): 1729-1666 (CO); 1552-1497 cm⁻¹ (C=N, C=C).
¹H-NMR (CDCl₃) 250 MHz: 1.26 (t, CH₃); 2.73 (t, CH₂); 3.79 (t, CH₂); 3.94 (m, CH₂); 4.14 (q, CH₂); 4.44 (m, CH₂); 5.38 (s, CH₂Ph); 7.35 (s, CH); 7.28-7.47 ppm (m, 5CH).

| | | |
|---|---|---|
| Massenspektrum: | Berechn. | 371.40 |
| | Gefund. | 372 (MH+). |

### 5-(2-Ethoxycarbony)-ethyl)-4,5,6,7-tetrahydro-4-oxo-pyrazolo[1,5-a]-pyrazin-2-carbonsäure (1.4)

Ein Gemisch aus 600 mg (1.61 mmol) 5-(2-Ethoxycarbonyl-ethyl)-4,5,6,7-tetrahydro-4-oxo-pyrazolo[1,5-a]-pyrazin-2-carbonsäure-benzylester (1.3) und 200 mg Palladium (5 % auf Aktivkohle) in 50 ml Ethanol wurde unter Wasserstoff gerührt, bis die Reaktion beendet war (ca. 4 h; DC: Kieselgel; Methanol/Methylenchlorid 5/95). Der Katalysator wurde abfiltriert und das Filtrat im Vakuum eingeengt. Ausbeute: 420 mg (92 %), amorphes Pulver

DC: R_{f} = 0.1 (Kieselgel; Methanol/Methylenchlorid 10/90)

IR (CHCl₃): 3510 (OH); 1724-1709-1667 (CO); 1553-1503-1495 cm⁻¹ (C=N, C=C).

¹H-NMR (CDCl₃) 250 MHz: 1.26 (t, CH₃); 2.75 (t, CH₂); 3.81 (t, CH₂); 3.98 (m, CH₂); 4.15 (q, CH₂); 4.48 (m, CH₂); 6.39 (bs, COOH); 7.41 ppm (s, CH).

| | | |
|---|---|---|
| Massenspektrum: | Berechn. | 281.27 |
| | Gefund. | 282 (MH+); 288 (MLi+). |

### (2S)-3-[5-(2-Ethoxycarbonyl-ethyl)-4,5,6,7-tetrahydro-4-oxo-pyrazolo[1,5-a]-pyrazin-2-carbonylamino]-2-[benzyloxycarbonylamino]-propionsäure-tert-butylester (1.5)

Ein Gemisch aus 420 mg (1.49 mmol) 5-(2-Ethoxycarbonyl-1-ethyl)-4,5,6,7-tetrahydro-4-oxo-pyrazolo[1,5-a]-pyrazin-2-carbonsäure (1.4), 0.82 ml (608 mg; 4.71 mmol) Diisopropylethylamin, 685 mg (2.13 mmol) O-Benzotriazol-1-yl-N,N,N',N'-tetramethyluronium-tetrafluoroborat und 414 mg (1.41 mmol) (2S)-3-Amino-2-[benzyloxycarbonylamino]-propionsäure-tert-butylester (1.6) in 50 ml Dimethylformamid wurde unter Stickstoffatmosphäre über Nacht gerührt. Das Reaktionsgemisch wurde mit 50 ml Essigsäureethylester verdünnt und die organische Phase mit 2 mal 10 ml 1N Salzsäure, 2 mal 30 ml gesättigter Natriumhydrogencarbonatlösung und 2 mal 30 ml gesättigter Natriumchloridlösung gewaschen. Nach Trocknen über Natriumsulfat und Filtration wurde das Lösungsmittel im Vakuum entfernt und der Rückstand über Kieselgel chromatographiert (Methylenchlorid bis Methanol/Methylenchlorid 2/98).
Ausbeute: 450 mg (53 %), Öl.

DC: R_{f} = 0.6 (Kieselgel; Methanol/Methylenchlorid 10/90)

[α]_{D}²⁰ = + 10° (CH₂Cl₂, c = 1.49)

IR (CHCl₃): 3420 (NH); 1722-1677 (CO); 1546-1506 cm⁻¹ (C=N, C=C, N-CO).

¹H-NMR (CDCl₃) 250 MHz: 1.26 (t, CH₃); 1.45 (s, tBu); 2.73 (t, CH₂); 3.79 (t, CH₂); 3.80-4.00 (m, 2CH₂); 4.14 (q, CH₂); 4.35 (m, CH₂); 4.42 (m, CH); 5.11 (s, CH₂Ph); 5.78 (d, NH); 7.13 (t, NH); 7.26-7.40 ppm (m, 6CH).

| | | |
|---|---|---|
| Massenspektrum: | Berechn. | 557.61 |
| | Gefund. | 558 (MH+); 564 (MLi+). |

### (2S)-3-Amino-2-benzyloxycarbonylaminopropionsäure-tert-butylester (1.6)

10 g (42 mmol) (2S)-3-Amino-2-benzyloxycarbonylamino-propionsäure wurden in einem Gemisch aus 100 ml Dioxan, 100 ml Isobutylen und 8 ml konz. H₂SO₄ 3 Tage bei 20 atm N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2 mal mit je 100 ml Diethylether extrahiert. Die vereinigten organischen Phasen wurden mit 2x100 ml H₂O gewaschen und über Na₂SO₄ getrocknet. Nach Entfernen des Lösungsmittels i. Vak. erhielt man 9.58 g (78 %) an (1.6) als blaßgelbes Öl.

### (2S)-3-[5-(2-Guanidinocarbonyl-ethyl)-4,5,6,7-tetrahydro-4-oxo-pyrazolo[1,5-a]-pyrazin-2-carbonylamino]-2-[benzyloxycarbonylamino]-propionsäure-tert-butylester (1.7)

300 mg (0.54 mmol) (2S)-3-[5-(2-Ethoxycarbonyl-ethyl)-4,5,6,7-tetrahydro-4-oxopyrazolo[1,5-a]-pyrazin-2-carbonylamino]-2-[benzyloxycarbonylamino]-propionsäure-tert-butylester (1.5) und 64 mg (1.08 mmol) Guanidin (Base) in 15 ml Dimethylformamid wurden unter Stickstoffatmosphäre über Nacht gerührt. Das Reaktionsgemisch wurde im Vakuum eingeengt und der Rückstand mehrmals über Kieselgel chromatographiert (Methylenchlorid bis Methanol/Methylenchlorid 15/95, dann Methanol/Methylenchlorid/Essigsäure/Wasser 15/85/2/2). Ausbeute: 40 mg (13 %), Öl.

DC: R_{f} = 0.4 (Kieselgel; Methanol/Methylenchlorid/Essigsäure/ Wasser 15/85/2/2)

¹H-NMR (DMSO-d₆) 300 MHz: 1.34 (s, tBu); 2.43 (t, CH₂); 3.40-3.75 (m, 2CH₂); 3.84 (m, CH₂); 4.14 (m, CH); 4.39 (m, CH₂); 5.04 (s, CH₂Ph); 6.94 (s, NH); 6.98 (s, CH); 7.35 (m, 5CH); 7.73 (d, NH); 8.35 ppm (m, NH).

| | | |
|---|---|---|
| Massenspektrum: | Berechn. | 570.61 |
| | Gefund. | 571 (MH+). |

### (2S)-3-[5-(2-Guanidinocarbonyl-ethyl)-4,5,6,7-tetrahydro-4-oxo-pyrazolo[1,5-a]-pyrazin-2-carbonylamino]-2-[benzyloxycarbonylamino]-propionsäure (1.8)

40 mg (0.07 mmol) (2S)-3-[5-(2-Guanidinocarbonyl-ethyl)-4,5,6,7-tetrahydro-4-oxo-pyrazolo-[1,5-a]-pyrazin-2-carbonylamino]-2-[benzyloxycarbonylamino]-propionsäure-tert-butylester (1.6) und 1.0 ml Trifluoroessigsäure in 5.0 ml Methylenchlorid wurden unter einer Stickstoffatmosphäre gerührt, bis die Reaktion beendet war (ca. 3 h; DC: Kieselgel; Methanol/Methylenchlorid/Essigsäure/ Wasser 15/85/2/2). Das Reaktionsgemisch wurde mit 5 ml Toluol verdünnt und im Vakuum eingeengt. Der Rückstand wurde in Essigsäureethylester aufgenommen, der Niederschlag abfiltriert und mehrmals mit Essigsäureethylester gewaschen und dann im Vakuum getrocknet. Ausbeute: 25 mg (70 %), amorphes Pulver.

DC: R_{f} = 0.20 (Kieselgel; Methanol/Methylenchlorid/Essigsäure/ Wasser 15/8512/2)

¹H-NMR (DMSO-d₆) 300 MHz: 2.60 (m, CH₂); 3.56 (m, CH₂); 3.69 (m, CH₂); 3.84 (m, CH₂); 4.38 (m, CH₂); 4.05 (m, CH); 5.02 (s, CH₂Ph); 6.98 (m, CH); 7.34 (m, 5CH); 7,70 (m, NH); 8,24 (breites m, NH); 8.50 (m, H).

| | | |
|---|---|---|
| Massenspektrum: | Berechn. | 514.50 |
| | Gefund. | 515 (MH+). |

### Beispiel 2

### (2S)-3-[5-(3-Guanidinocarbonyl-propyl)-4,5,6,7-tetrahydro-4-oxo-pyrazolo[1,5-a]-pyrazin-2-carbonylamino]-2-[benzyloxycarbonylamino]-propionsäure (2.7)

Die Synthese erfolgte nach folgender Reaktionssequenz:

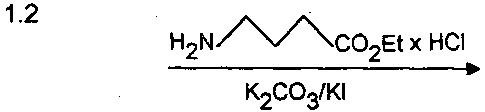

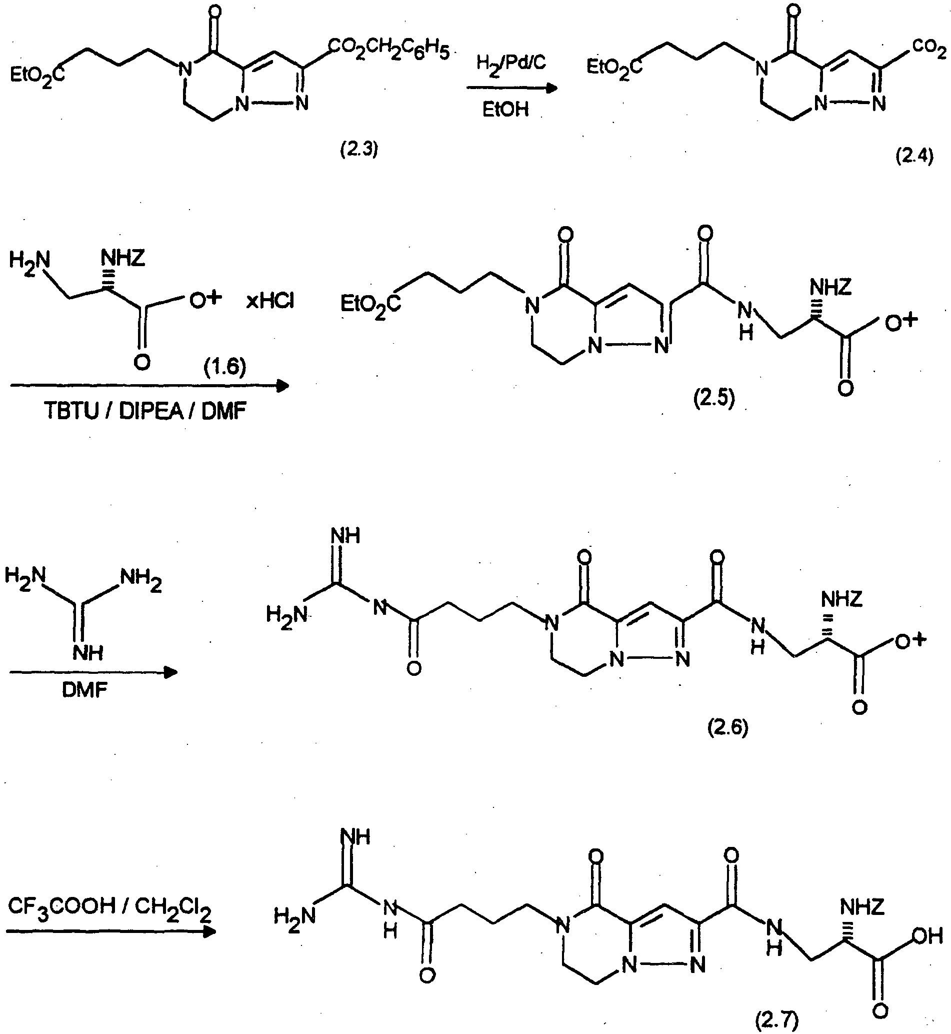

### 5-(3-Ethoxycarbonyl-propyl)-4,5,6,7-tetrahydro-4-oxo-pyrazolo[1,5-a]-pyrazin-2-carbonsäure-dibenzylester (2.3)

500 mg (1.13 mmol) 1-(2-Bromo-ethyl)-pyrazol-3,5-dicarbonsäure-dibenzylester (1.2; siehe Beispiel 1), 315 mg (2.28 mmol) Kaliumcarbonat, 20 mg Kaliumiodid und 206 mg (1.23 mmol) 4-Aminobuttersäureethylester-hydrochlorid in 50 ml Acetonitril wurden in einer Stickstoffatmosphäre unter Rückfluß erhitzt, bis die Reaktion beendet war (ca. 40 h; DC: Kieselgel; Methanol/Methylenchlorid 10/90). Das Reaktionsgemisch wurde filtriert und im Vakuum eingeengt. Der Rückstand wurde über Kieselgel chromatographiert (Methylenchlorid, dann Methanol/Methylenchlorid 1/99). Ausbeute: 240 mg (55 %), Öl.

DC: R_{f} = 0.6 (Kieselgel; Methanol/Methylenchlorid 5/95)

IR (CHCl₃): 1729-1667 (CO); 1552-1496 cm-1 (C=N, C=C).

¹H-NMR (CDCl₃) 250 MHz: 1.24 (t, CH₃); 1.96 (m, CH₂); 2.40 (t, CH₂); 3.61 (t, CH₂); 3.81 (m, CH₂); 4.10 (q, CH₂); 4.48 (m, CH₂); 5.40 (s, CH₂Ph); 7.36 (s, CH); 7.30-7.48 ppm (m, 5CH).

| | | |
|---|---|---|
| Massenspektrum: | Berechn. | 385.42 |
| | Gefund. | 386 (MH+), 392 (MLi+) |

### 5-(3-Ethoxycarbonyl-propyl)-4,5,6,7-tetrahydro-4-oxo-pyrazolo[1,5-a]-pyrazin-2-carbonsäure (2.4)

700 mg (1.82 mmol) 5-(3-Ethoxycarbonyl-propyl)-4,5,6,7-tetrahydro-4-oxo-pyrazolo[1,5-a]-pyrazin-2-carbonsäure-benzylester (2.3) und 217 mg Palladium (5 % auf Aktivkohle) in 50 ml Ethanol wurden unter Wasserstoff gerührt, bis die Reaktion beendet war (ca. 4 h; DC: Kieselgel; Methanol/Methylenchlorid 10/90). Der Katalysator wurde abfiltriert und das Filtrat im Vakuum eingeengt. Ausbeute: 240 mg (75 %), amorphes Pulver.
Schmelzpunkt: 175°C (Koflerbank)

DC: R_{f} = 0.1 (Kieselgel; Methanol/Methylenchlorid 10/90)

IR (Nujol): 1715-1625 (CO); 1580-1554 cm⁻¹ (C=N, C=C).

¹H-NMR (DMSO-d₆) 300 MHz: 1.16 (t, CH₃); 1.81 (m, CH₂); 2.34 (t, CH₂); 3,48 (t, CH₂); 3.81 (m, CH₂); 4.03 (q, CH₂); 4.45 (m, CH₂); 7.03 (s, CH); 13.0 ppm (s, COOH).

| | | |
|---|---|---|
| Massenspektrum: | Berechn. | 295.30 |
| | Gefund. | 296 (MH+), 318 (MNa+). |

### (2S)-3-[5-(3-Ethoxycarbonyl-propyl)-4,5,6,7-tetrahydro-4-oxo-pyrazolo[1,5-a]-pyrazin-2-carbonylamino]-2-[benzyloxycarbonylamino]-propionsäure-tert-butylester (2.5)

400 mg (1.35 mmol) 5-(3-Ethoxycarbonyl-propyl)-4,5,6,7-tetrahydro-4-oxo-pyrazolo[1,5-a]-pyrazin-2-carbonsäure (2.4), 0.70 ml (519 mg; 4.01 mmol) Diisopropylethylamin, 589 mg (1.83 mmol) O-Benzotriazol-1-yl-N,N,N',N'-tetramethyluronium-tetrafluoroborat und 356 mg (1.21 mmol) (2S)-3-Amino-2-[benzyloxycarbonylamino]-propionsäure-tert-butylester (1.6) (zur Synthese vgl. Beispiel 1) in 50 ml Dimethylformamid wurden unter Stickstoffatmosphäre 48 h gerührt. Das Reaktionsgemisch wurde mit 50 ml Essigsäureethylester verdünnt und die organische Phase mit 2 mal 10 ml 1 N Salzsäure, 2 mal 20 ml gesättigte Natriumhydrogencarbonatlösung und 2 mal 30 ml gesättigte Natriumchloridlösung gewaschen. Nach Trocknen über Natriumsulfat wurde im Vakuum eingeengt und der Rückstand über Kieselgel chromatographiert (Methylenchlorid bis Methanol/Methylenchlorid 2/98). Ausbeute: 440 mg (57 %), Öl.

DC: R_{f} = 0.2 (Kieselgel; Methanol/Methylenchlorid 5/95)

[α]_{D}²⁰ = + 5° (CH₂Cl₂, c = 0.58),

IR (CHCl₃): 3420 (NH); 1722-1676 (CO); 1547-1505 cm⁻¹ (C=N, C=C, N-CO).

¹H-NMR (CDCl₃) 250 MHz: 1.23 (t, CH₃); 1.45 (s, tBu); 1.96 (m, CH₂); 2.39 (t, CH₂); 3.60 (t, CH₂); 3.78 (t, CH₂); 3.87 (m, CH₂); 4.10 (q, CH₂); 4.32 (t, CH₂); 4.40 (m, CH); 5.14 (s, CH₂Ph); 5.81 (d, NH); 7.17 (t, NH); 7.32 ppm (m, 6CH).

| | | |
|---|---|---|
| Massenspektrum: | Berechn. | 571.64 |
| | Gefund. | 572 (MH+) - 610 (MK+). |

### (2S)-3-[5-(3-Guanidinocarbonyl-propyl)-4,5,6,7-tetrahydro-4-oxo-pyrazolo[1,5-a]-pyrazin-2-carbonylamino]-2-[benzyloxycarbonylamino]-propionsäure-tert-butylester (2.6)

200 mg (0.35 mmol) (2S)-3-[5-(3-Ethoxycarbonyl-propyl)-4,5,6,7-tetrahydro-4-oxo-pyrazolo[1,5-a]-pyrazin-2-carbonylamino-2-[benzoyloxycarbonylamino]-propionsäure-tert-butylester (2.5) und 42 mg (0.71 mmol) Guanidin (Base) in 10 ml Dimethylformamid wurden unter Stickstoffatmosphäre über Nacht bei Raumtemperatur gerührt. Das Reaktionsgemisch wurde im Vakuum eingeengt und der Rückstand über Kieselgel chromatographiert (Methylenchlorid bis Methanol/Methylenchlorid 15/85, dann Methanol/Methylenchlorid/Essigsäure/Wasser 15/85/2/2). Ausbeute: 60 mg (29 %), Öl.

DC: R_{f} = 0.3 (Kieselgel; Methanol/Methylenchlorid/Essigsäure/Wasser 15/85/2/2).

¹H-NMR (DMSO-d₆) 300 MHz: 1.33 (s, tBu); 1.78 (m, CH₂); 2.19 (t, CH₂); 3.30-3.70 (m, 2CH₂); 3.83 (m, CH₂); 4.42 (m, CH₂); 4.13 (m, CH); 5.03 (s, CH₂Ph); 6.99 (m, CH); 7.36 (m, 5CH); 7.75 (d, NH); 8.35 ppm (m, NH).

| | | |
|---|---|---|
| Massenspektrum: | Berechn. | 584.64 |
| | Gefund. | 585 (MH+), 607 (MNa+). |

### (2S)-3-[5-(3-Guanidinocarbonyl-propyl)-4,5,6,7-tetrahydro-4-oxo-pyrazolo[1,5-a]-pyrazin-2-carbonylamino]-2-[benzyloxycarbonylamino]-propionsäure (2.7)

35 mg (0.06 mmol) (2S)-3-[5-(3-Guanidinocarbonyl-propyl)-4,5,6,7-tetrahydro-4-oxo-pyrazolo[1,5-a]-pyrazin-2-carbonylamino]-2-[benzoyloxycarbonylamino]-propionsäure-tert-butylester (2.6) und 0.5 ml Trifluoroessigsäure in 1.5 ml Methylenchlorid wurden unter Stickstoffatmosphäre gerührt, bis die Reaktion beendet ist (ca. 1 h, DC: Kieselgel; Methanol/Methylenchlorid/Essigsäure/ Wasser 15/85/2/2). Das Reaktionsgemisch wurde mit 5 ml Toluol verdünnt und im Vakuum eingeengt. Der Rückstand wurde mit einem Gemisch aus Diethylether/ Methylenchlorid verdünnt, der Niederschlag filtriert und mehrmals mit Diethylether/Methylenchlorid gewaschen und dann unter Vakuum getrocknet. Ausbeute: 30 mg (95 %), amorphes Pulver.

DC: R_{f} = 0.15 (Kieselgel; Methanol/Methylenchlorid/Essigsäure/ Wasser 15/85/212).

¹H-NMR (DMSO-d₆) 300 MHz: 1.84 (m, CH₂); 2.45 (m, CH₂); 3.51 (m, 2CH₂); 3.81 (m, CH₂); 4.09 (m, CH); 4.44 (m, CH₂); 5.02 (s, CH₂Ph); 7.01 (m, CH); 7.34 (m, 5H); 8.24 ppm (breites m, H).

| | | |
|---|---|---|
| Massenspektrum: | Berechn. | 528.53 |
| | Gefund. | 529 (MH+), 551 (MNa+). |

### Beispiel 3

### 3-[2-(Guanidinocarbonylmethyl)-2,3-dihydro-3-oxo-[1,2,4]triazolo[4,3-a]pyridin-6-carbonylamino]-2-[(phenylmethoxy)carbonylamino]propionsäure (3.5)

Die Synthese wurde nach den folgenden Vorschriften durchgeführt.

2-(Ethoxycarbonylmethyl)-2,3-dihydro-3-oxo-[1,2,4]triazolo[4,3-a]pyridin-6-carbonsäure-tert-butylester (3.1)

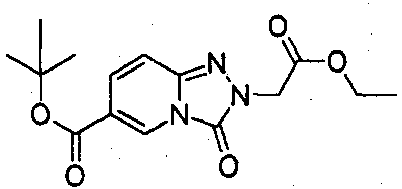

Eine Mischung von 2,3-Dihydro-3-oxo-[1,2,4]triazolo[4,3-a]pyridin-6-carbonsäure-tert-butylester [hergestellt nach WO 94-18981] (350 mg, 1.49 mmol), Cesiumcarbonat (485 mg, 1.49 mmol) und 2-Bromessigsäureethylester (0.26 ml, 2.26 mmol) in Acetonitril (50 ml) wurde 1 h unter Rückfluß erhitzt. Nach dem Abkühlen auf Raumtemperatur wurde der Niederschlag abfiltiert und das Filtrat unter vermindertem Druck zur Trockne eingeengt. Der Rückstand wurde chromatographiert (Kieselgel; Elution mit Chloroform/Essigester 80/20 v/v). Durch Umkristallisation des erhaltenen Feststoffes aus Diisopropylether wurden hellgelbe Kristalle erhalten (330 mg, 68%).

Schmelzpunkt: 108°C.

DC: R_{f} = 0.60 (Kieselgel; Chloroform/Essigester 80/20 v/v).

IR (CHCl₃): 1751, 1734, 1718 (C=O), 1643, 1559, 1539 cm⁻¹ (C=N + C=C).

¹H-NMR (CDCl₃) 250 MHz: 1.30 (t, 3H, CH₃), 1.59 (s, 9H, t-Bu), 4.26 (q, 2H, CH₂), 4.77 (s, 2H, CH₂), 7.08 (dd, 1H, arom.), 7.59 (dd, 1H, arom.), 8.47 ppm (t, 1 H, arom.).

| | | | | |
|---|---|---|---|---|
| CHN-Analyse: | Berechn. | C 56.07; | H 5.96; | N 13.08. |
| | Gefund. | C 56.3; | H 6.1; | N 12.8. |

### 2-(Ethoxycarbonylmethyl)-2,3-dihydro-3-oxo-[1,2,4]triazolo[4,3-a]pyridin-6-carbonsäure (3.2)

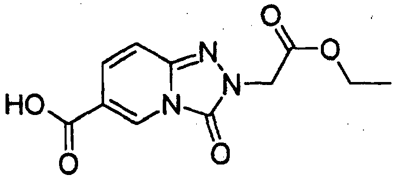

Zu einer auf 0°C gekühlten Lösung von 2-(Ethoxycarbonylmethyl)-2,3-dihydro-3-oxo-[1,2,4]triazolo[4,3-a]pyhdin-6-carbonsäure-tert-butylester (300 mg, 0.93 mmol) in Methylenchlorid (5 ml) wurde Trifluoressigsäure (5 ml) gegeben. Die Mischung wurde 4 h bei 0°C gerührt und dann unter vermindertem Druck zur Trockne eingeengt. Durch Umkristallisation des festen Rückstandes aus Diisopropylether/Isopropanol wurden Kristalle erhalten (155 mg, 63%).

Schmelzpunkt: 188°C.

DC: R_{f} = 0.20 (Kieselgel; Methylenchlorid/Methanol 80/20 v/v).

IR (CHCl₃): 1743,1734, 1700 (C=O), 1641, 1558, 1536 cm⁻¹ (C=N + C=C).

¹H-NMR (DMSO-d₆) 250 MHz: 1.21 (t, 3H, CH₃), 4.17 (q, 2H, CH₂), 4.84 (s, 2H, CH₂), 7.30 (dd, 1 H, arom.), 7.55 (dd, 1 H, arom.), 8.29 (dd, 1 H, arom.), 13.44 ppm (breites s, 1H, COOH).

| | | | | |
|---|---|---|---|---|
| CHN-Analyse: | Berechn. | C 49.81; | H 4.18; | N,15.84. |
| | Gefund. | C 49.7; | H 4.0; | N 15.7. |

### 3-[2-(Ethoxycarbonylmethyl)-2,3-dihydro-3-oxo-[1,2,4]triazolo[4,3-a]pyridin-6-carbonylamino]-2-[(phenylmethoxy)carbonylamino]propionsäure-tert-butylester (3.3)

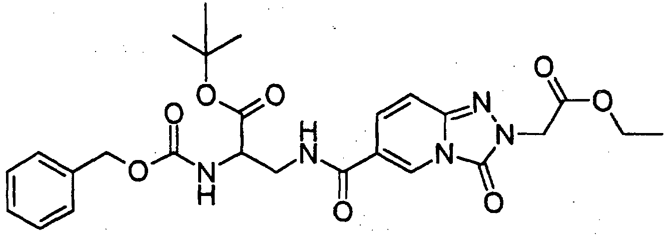

Zu einer LÖsung von 2-(Ethoxycarbonylmethyl)-2,3-dihydro-3-oxo-[1,2,4]triazolo(4,3-a]pyridin-6-carbonsäure (220 mg, 0.83 mmol) in trockenem Dimethylformamid (5 ml) wurden nacheinander N,N-Diisopropylethylamin (0.435 ml, 2.5 mmol), O-Benzotriazol-1-yl-N,N,N',N'-tetramethyluronium-tetrafluoroborat (362 mg, 1.13 mmol) und 3-Amino-2-(benzyloxycarbonylamino)propionsäure-tert-butylester (244 mg, 0.83 mmol) gegeben und die Mischung wurde über Nacht bei Raumtemperatur unter Inertgasatmosphäre gerührt. Nach Zugabe von Essigester (150ml) wurde die organische Phase mit 1 N Salzsäure (2x50 ml), gesättigter Natriumhydrogencarbonatlösung (2x50ml) und Kochsalzlösung (1x50 ml) gewaschen, über Magnesiumsulfat getrocknet und unter vermindertem Druck zur Trockne eingeengt. Durch Chromatographie (Kieselgel; Elution mit Essigester/Triethylamin 98/2 v/v) des Rückstandes wurde ein hellgelbes Öl erhalten (170 mg, 38%).

DC: R_{f} = 0.70 (Kieselgel; Essigester/Triethylamin 98/2 v/v).

IR (CHCl₃): 3414 (NH), 1729, 1672 (C=O), 1642, 1630,1560, 1532, 1506 cm⁻¹ (C=C+ C=N + Amid).

¹H-NMR (CDCl₃) 250 MHz: 1.30 (t, 3H, CH₃), 1.48 (s, 9H, t-Bu), 3.79 (m, 2H, CH₂), 4.27 (q, 2H, CH₂), 4.45 (m, 1 H, CH), 4.77 (s, 2H, CH₂), 5.14 (s, 2H, CH₂Ph), 5.89 (d, 1 H, NH), 7.08 (dd, 1 H, arom.), 7.33 (m, 6H, arom.+ NH), 7.41 (d, 1 H, arom.), 8.31 ppm (s, 1 H, arom.).

| | | | | |
|---|---|---|---|---|
| CHN-Analyse: | Berechn. | C 57.66; | H 5.77; | N 12.93. |
| | Gefund. | C 57.6; | H 5.9; | N 12.6. |

### 3-[2-(Guanidinocarbonylmethyl)-2,3-dihydro-3-oxo-[1,2,4]triazolo[4,3-a]pyridin-6-carbonylamino]-2-[(phenylmethoxy)carbonylamino]propionsäure-tert-butylester (3.4)

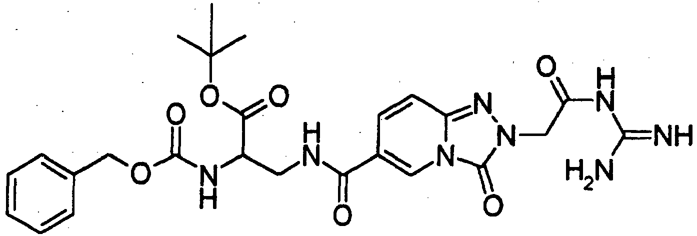

Eine Mischung von 3-[2-(Ethoxycarbonylmethyl)-2,3-dihydro-3-oxo-[1,2,4]triazolo[4,3-a]pyridin-6-carbonylaminol-2-[(phenylmethoxy)carbonylamino]propionsäure-tert-butylester (170 mg, 0.31 mmol) und Guanidin (Base) (30 mg, 0.51 mmol) in trockenem Tetrahydrofuran (10 ml) und t-Butanol (0.5 ml) wurde über Nacht bei Raumtemperatur unter Inertgasatmosphäre gerührt, dann unter vermindertem Druck zur Trockne eingeengt. Durch Chromatographie (Kieselgel; Elution mit Methylenchlorid/Methanol 85/15 v/v) des Rückstandes wurde ein farbloses Öl erhalten (100 mg, 58%).

DC: R_{f} = 0.40 (Kieselgel; Methylenchlorid/Methanol 85/15 v/v).

IR (CHCl₃): 3505, 3405, 3310 (NH/NH₂), 1731, 1714 (C=O), 1667, 1627, 1607, 1532 cm⁻¹ (C=O + C=N + C=C + NH/NH₂).

¹H-NMR (DMSO-d₆) 300 MHz: 1.35 (s, 9H, tBu), 3.58 (m, 2H, CH₂), 4.22 (m, 1 H, CH), 4.46 (s, 2H, CH₂), 5.05 (m, 2H, CH₂Ph), 6.66 (breites s, NH), 7.27 (d, 1 H, arom.), 7.34 (m, 5H, arom.), 7.52 (dd, 1 H, arom.), 7.69 (d, 1 H, NH), 7.73 (breites s, NH), 8.47 (breites s, 1 H, arom.), 8.67 ppm (t, 1 H, NH).

Massenspektrum: 555 (MH+), 577 (MNa+).

| | | | | |
|---|---|---|---|---|
| CHN-Analyse: | Berechn. | C 54.15; | H 5.45; | N 20.21. |
| | Gefund. | C 52.5; | H 5.2; | N 19.0. |

### 3-[2-(Guanidinocarbonylmethyl)-2,3-dihydro-3-oxo-[1,2,4]triazolo[4,3-a]pyridin-6-carbonylamino]-2-[(phenylmethoxy)carbonylamino]propionsäure (3.5)

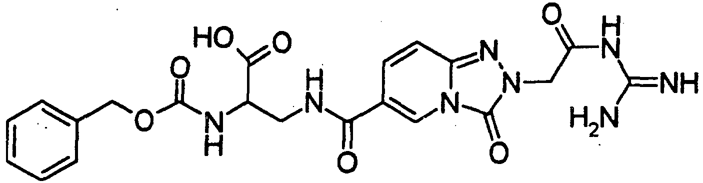

Zu einer auf 0°C gekühlten Lösung von 3-[2-(Guanidinocarbonylmethyl)-2,3-dihydro-3-oxo-[1,2,4]triazolo[4,3-a]pyridin-6-carbonylamino]-2-[(phenylmethoxy)carbonylamino]propionsäure-tert-butylester (95 mg, 0.17 mmol) in Methylenchlorid (5 ml) wurde Trifluoressigsäure (2 ml) gegeben. Nachdem die Mischung auf Raumtemperatur gekommen war, wurde weitere 3 h gerührt, dann mit Toluol (20 ml) versetzt und unter vermindertem Druck zur Trockne eingeengt. Durch Chromatographie (Kieselgel; Elution mit Methylenchlorid/Methanol/Ammoniumhydroxid 70/30/4 v/v/v) des Rückstandes wurde ein blaßgelbes Pulver erhalten (20 mg, 24%).

Schmelzpunkt: > 250°C Zers.

DC: R_{f} = 0.30 (Kieselgel; Methylenchlorid/Methanol/Ammoniumhydroxid 70/30/4 v/v/v).

IR (Nujol): 3374 (NH/OH), 1710 (C=O), 1654 (C=O + C=N), 1632, 1525 cm⁻¹ (arom. + Amid).

¹H-NMR (DMSO-d₆) 300 MHz: 3.59 (m, 2H, CH₂), 4.23 (q, 1H, CH), 4.46 (s, 2H, CH₂), 5.03 (AB, 2H, CH₂Ph), 6.71 (breites s, 1 H, NH), 7.09 (breites s, NH), 7.26 (d, 1 H, arom.), 7.33 (breites s, 5H, arom.), 7.53 (breites d, 1 H, arom.), 7.78 (breites s, NH), 8.47 (breites s, 1H, arom.), 8.72 ppm (t, NH).

Massenspektrum: 499 (MH+).

| | | | | |
|---|---|---|---|---|
| CHN-Analyse: | Berechn. | C 50.60; | H 4.45; | N 22.48. |
| | Gefund. | C 48.1; | H 4.6; | N 19.0. |

### Beispiel 4

### 2-(Benzyloxycarbonylamino)-3-[[2-(2-(guanidylcarbonyl)-1-ethyl)-5-benzimidazolyl]carbonylamino]propionsäure (4.5)

Die Synthese wurde nach den folgenden Vorschriften durchgeführt.

### 3-[5-Carboxy-2-benzimidazolyl]propionsäuremethylester (4.1)

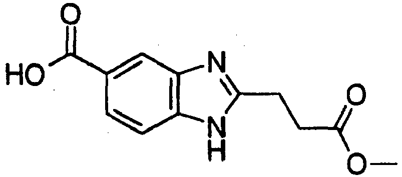

Zu einer Mischung von 3,4-Diaminobenzoesäure (3.0 g, 20 mmol) und Triethylamin (5.5 ml, 40 mmol) in Tetrahydrofuran (400 ml) wurde bei Raumtemperatur und unter Inertgasatmosphäre innerhalb von 20 min eine Lösung von 3-Carbomethoxypropionylchlorid (2.4 ml, 20 mmol) in Tetrahydrofuran (100 ml) gegeben. Die braune Mischung wurde bei Raumtemperatur 24 h gerührt, der gebildete Niederschlag abfiltriert und das Filtrat unter vermindertem Druck zur Trockne eingeengt. Zum Rückstand wurde Essigsäure (300 ml) gegeben und die Mischung 5 h unter Rückfluß erhitzt. Die Mischung wurde dann unter vermindertem Druck zur Trockne eingeengt und der Rückstand chromatographiert (Kieselgel; Elution mit Methylenchlorid/Essigester 50/50 v/v, dann Methylenchlorid/Essigester/Methanol 50/50/5 v/v/v und schließlich Methylenchlorid/Essigester/Methanol/Essigsäure 50/50/5/2 v/v/v/v). Es wurde ein amorpher Feststoff erhalten (2.6 g, 52%).

DC: R_{f} = 0.05 (Kieselgel; Essigester).

IR (Nujol): 3250 (OH,NH), 1717,1685 (C=O), 1624, 1593, 1542, 1480 cm⁻¹ (C=N + arom.).

¹H-NMR (DMSO-d₆) 250 MHz: 2.91 (m, 2H, CH₂), 3.12 (m, 2H, CH₂), 3.61 (s, 3H, CH₃), 7.52 (d, 1 H, arom.), 7.77 (dd, 1 H, arom.), 8.06 (breites s, 1 H, arom.), 12.60 ppm (breites m, 2H, NH + OH).

Massenspektrum: 248 (M+).

### 3-(5-(2-(Benzyloxycarbonylamino)-2-t-butoxycarbonyl-1-ethylaminocarbonyl)-2-benzimidazolyl]propionsäuremethylester (4.2)

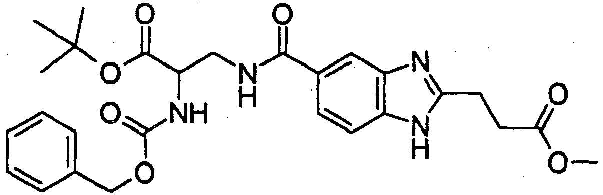

Eine Mischung von 3-[5-Carboxy-2-benzimidazolyl]propionsäuremethylester (1.7 g, 6.8 mmol), 3-Amino-2-(benzyloxycarbonylamino)propionsäure-tert-butylester (2.6 g, 8.8 mmol), 1-Hydroxybenzotriazol (1.1 g, 8.1 mmol), 1-(3-Dimethylaminopropyl)-3-ethylcarbodimid-hydrochlorid (1.7 g, 8.8 mmol) und N-Methylmorpholin (1.5 ml, 13.6 mmol) in trockenem Dimethylformamid (50 ml) wurde bei Raumtemperatur unter Inertgasatmosphäre 48 h gerührt. Die Mischung wurde unter vermindertem Druck zur Trockne eingeengt und der Rückstand chromatographiert (Kieselgel; Essigester). Es wurde ein amorpher Feststoff erhalten (2.0 g, 55%).

DC: R_{f} = 0.40 (Kieselgel; Essigester).

IR (CHCl₃): 3445, 3413 (NH), 1722, 1661 (C=O), 1626, 1600, 1580, 1536 (arom.), 1440 (COOMe), 1370 cm⁻¹ (COOtBu).

¹H-NMR (CDCl₃) 300 MHz: 1.45 (s, 9H, t-Bu), 2.90 (m, 2H, CH₂), 3.23 (m, 2H, CH₂), 3.72 (s, 3H, CH₃), 3.87 (t, 2H, CH₂), 4.43 (q, 1H, CH), 5.10 (breites s, 2H, CH₂Ph), 6.05 (d, 1 H, NH), 7.05 (t, 1H, NH), 7.29 (m, 5H, arom.). 7.54 (m, 1 H, arom.), 7.59 (m, 1 H, arom.), 7.98 ppm (breites s, 1 H, arom.).

Massenspektrum: 525 (MH+), 547 (MNa+).

### 3-[5-[2-(Benzyloxycarbonylamino)-2-t-butoxycarbonyl-1-ethylaminocarbonyl]-1-t-butoxycarbonyl-2-benzimidazolyl]propionsäuremethylester und 3-[5-[2-(Benzyloxycarbonylamino)-2-t-butoxycarbonyl-1-ethylaminocarbonyl]-3-t-butoxycarbonyl-2-benzimidazolyl]propionsäuremethylester (4.3)

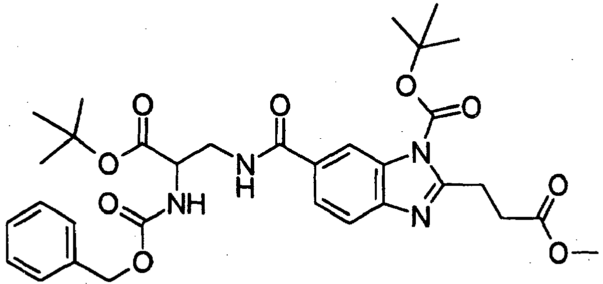

und/oder

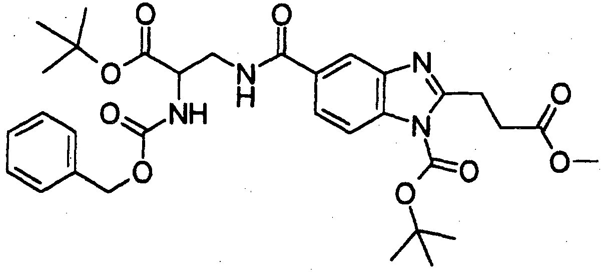

Zu einer Mischung von 3-[5-[2-(Benzyloxycarbonylamino)-2-t-butoxycarbonyl-1-ethylaminocarbonyl]-2-benzimidazolyl]propionsäuremethylester (965 mg, 1.84 mmol), Di-tert-butyldicarbonat (405 mg, 1.85 mmol) und 4-Dimethylaminopyridin (224 mg, 1.83 mmol) in Methylenchlorid (80 ml) wurde bei Raumtemperatur unter Inertgasatmosphäre Triethylamin (260 µl, 1.86 mmol) gegeben. Nach 1/2 h Rühren wurde Diethylether (100 ml) und Wasser (50 ml) zugegeben. Die organische Phase wurde abgetrennt, über Magnesiumsulfat getrocknet und unter vermindertem Druck zur Trockne eingeengt. Durch Chromatographie (Kieselgel; Elution mit einem Gradienten Methylenchlorid/Diethylether von 100/0 bis 0/100) wurde ein weißer amorpher Feststoff erhalten (790 mg, 68%).

DC: R_{f} = 0.90 (Kieselgel; Essigester).

IR (CHCl₃): 3416 (NH), 1736, 1661 (C=O), 1620, 1588, 1507 cm⁻¹ (arom.+ Amid)

¹H-NMR (DMSO-d₆) 300 MHz: 1.30 und 1.36 (2s, 2x9H, tBu), 2.91 (t, 2H, CH₂), 3.40 (td, 2H, CH₂), 3.62 (sd, 3H, CH₃), 3.69 und 3.96 (2m, 2H, CH₂), 5.03-5.20 (m, 3H, CH + CH₂Ph), 7.30-7.34 (m, 5H, arom.), 7.68 und 7.93 (2d, 1 H, arom.), 7.75 und 7.82 (2d, 1 H, arom.), 8.10 und 8.40 (2s, 1 H, arom.), 8.75 ppm (d, 1H, NH).

Massenspektrum: 625 (MH+), 647 (MNa+).

### 2-(Benzyloxycarbonylamino)-3-[[2-(2-(guanidylcarbonyl)-1-ethyl)-5-benzimidazolyl]carbonylamino]propionsäure-tert-butylester (4.4)

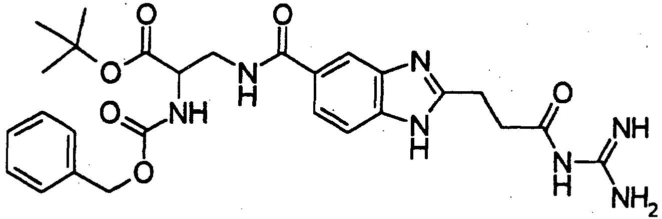

Eine Mischung von 3-[5-[2-(Benzyloxycarbonylamino)-2-t-butoxycarbonyl-1-ethylaminocarbonyl]-1 (und 3)-t-butoxycarbonyl-2-benzimidazolyl]propionsäuremethylester (790 mg, 1.26 mmol) und Guanidin (Base) (780 mg, 13 mmol) in trockenem Dimethylformamid (30 ml) wurde bei Raumtemperatur unter Inertgasatmosphäre 1 h gerührt. Die Mischung wurde unter vermindertem Druck zur Trockne eingeengt und der Rückstand chromatographiert (Kieselgel; Elution mit einem Gradienten Methylenchlorid/Methanot/Wasser/Essigsäure von 90/10/1/1 bis 85/15/2/2 und schließlich 70/30/6/3 v/v/v/v). Es wurde ein weißes Pulver erhalten (860 mg). Zu diesem Feststoff wurde Methanol (6 ml) gegeben, die Lösung filtriert und das Filtrat in eine Mischung von Essigester/Diethylether/Pentan (600 ml, 1/1/1 v/v/v) gegossen. Der Niederschlag wurde abfiltriert, mit Pentan gewaschen und im Vakuum getrocknet. Es wurde ein amorpher Feststoff erhalten (470 mg, 68%).

DC: R_{f} = 0.28 (Kieselgel; Methylenchlorid/Methanol/Wasser/Essigsäure 85/15/2/2 v/v/v/v).

IR (Nujol): 1730, 1701 (C=O), 1623, 1540 cm⁻¹ (C=O + C=N + arom. + Amid).

¹H-NMR (DMSO-d₆) 300 MHz: 1.33 und 1.40 (2s, 9H, t-Bu), 2.92 (t, 2H, CH₂), 3.09 (t, 2H, CH₂), 3.58 und 3.70 (m, 2H, CH₂), 4.24 (q, 1 H, CH), 5.05 (s, 2H, CH₂Ph), 7.11 (breites s, NH), 7.32 (breites m, NH), 7.35 (s, 5H, arom.), 7.48 (d, 1 H, arom.), 7.62 (breites d, 1H, arom.), 7.71 (breites d, NH), 7.90 (breites s, 1 H, arom.), 8.42 ppm (breites m, NH).

Massenspektrum: 552 (MH+).

### 2-(Benzyloxycarbonylamino)-3-[[2-(2-(guanidylcarbonyl)-1-ethyl)-5-benzimidazolyl]carbonylamino]propionsäure (4.5)

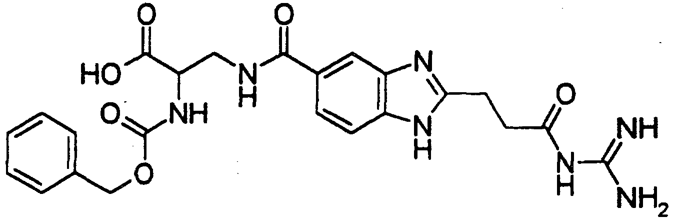

Zu einer Suspension von 2-(Benzyloxycarbonylamino)-3-[[2-(2-(guanidylcarbonyl)-1-ethyl)-5-benzimidazolyl]carbonylamino]propionsäure-tert-butylester (230 mg, 0.42 mmol) in Methylenchlorid (10 ml) wurde tropfenweise Trifluoressigsäure (2 ml) gegeben. Die Mischung wurde 2 h bei Raumtemperatur gerührt, mit Toluol (50 ml) versetzt, unter vermindertem Druck zur Trockne eingeengt und der Rückstand in Methanol (1.5 ml) gelöst. Die Lösung wurde in eine Mischung von Diethylether/Essigester (200 ml, 1/1) gegossen, der Niederschlag isoliert und diese Fällung nochmals wiederholt. Nach Abfiltrieren und Trockenen des Niederschlags im Vakuum wurde ein amorphes Pulver erhalten (85 mg, 41 %).

DC: R_{f} = 0.05 (Kieselgel; Methylenchlorid/Methanol/Wasser/Essigsäure 85/15/2/2 v/v/v/v).

¹H-NMR (DMSO-d₆) 300 MHz: 3.04 (m, 2H, CH₂), 3.14 (m, 2H, CH₂), 3.62 (m, 2H, CH₂), 4.24 (m, 1 H, CH), 5.02 (breites s, 2H, CH₂Ph), 7.32 (m, 5H, arom.), 7.51 (m, 2H, arom.+ NH), 7.65 (d, 1H, arom.), 7.99 (breites s, 1 H, arom.), 8.20-8.70 (breites m, 4H, NH), 12.53 ppm (m, 2H, NH + COOH).

Massenspektrum: 518 (MNa+), 496 (MH+).

### Beispiel 5

### 3-[2-(2-Guanidinocarbonyl-1-ethyl)-2,3-dihydro-3-oxo-[1,2,4]triazolo[4,3-a]pyridin-6-carbonylamino]-2-[(phenylmethoxy)carbonylamino]propionsäure (5.5)

Die Synthese wurde nach den folgenden Vorschriften durchgeführt.

### 2-(2-Ethoxycarbonyl-1-ethyl)-2,3-dihydro-3-oxo-[1,2,4]triazolo[4,3-a]pyridin-6-carbonsäure-tert-butylester (5.1)

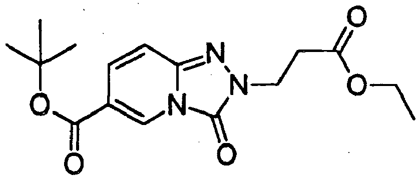

Eine Mischung von 2,3-Dihydro-3-oxo-[1 ,2,4]triazolo[4,3-a]pyridin-6-carbonsäure-tert-butylester [hergestellt nach WO 94-18981] (500 mg, 2.1 mmol), Cesiumcarbonat (1.38 g, 4.2 mmol) und 3-Brompropionsäureethylester (0.41 ml, 3.2 mmol) in Acetonitril (50 ml) wurde 8 h unter Rückfluß erhitzt und über Nacht bei Raumtemperatur gerührt. Der Niederschlag wurde abfiltriert und das Filtrat unter vermindertem Druck zur Trockne eingeengt. Durch Chromatographie des Rückstandes (Kieselgel; Elution mit Methylenchlorid/Essigester 80/20 v/v) wurde ein hellgelbes Öl erhalten (500 mg, 71%).

DC: R_{f} = 0.71 (Kieselgel; Chloroform/Essigester 60/40 v/v).

IR (CHCl₃): 1717 (C=O), 1641, 1557, 1535 cm⁻¹ (C=N + C=C).

¹H-NMR (CDCl₃) 250 MHz: 1.25 (t, 3H, CH₃), 1.58 (s, 9H, t-Bu), 2.87 (t, 2H, CH₂), 4.16 (q, 2H, CH₂), 4.31 (t, 2H, CH₂), 7.06 (dd, 1 H, arom.), 7.56 (dd, 1 H, arom.), 8.45 ppm (t, 1 H, arom.).

| | | | | |
|---|---|---|---|---|
| CHN-Analyse: | Berechn. | C 57.30; | H 6.31; | N 12.53. |
| | Gefund. | C 57.3; | H 6.2; | N 12.4. |

### 2-(2-Ethoxycarbonyl-1-ethyl)-2,3-dihydro-3-oxo-[1,2,4]triazolo[4,3-a]pyridin-6-carbonsäure (5.2)

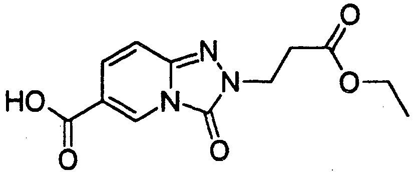

Zu einer auf 0°C gekühlten Lösung von 2-(2-Ethoxycarbonyl-1-ethyl)-2,3-dihydro-3-oxo-[1,2,4]triazolo[4,3-a]pyridin-6-carbonsäure-tert-butylester (1.3 g, 3.9 mmol) in Methylenchlorid (20 ml) wurde Trifluoressigsäure (20 ml) zugegeben. Die Mischung wurde 1 h bei 0°C und dann 24 h bei Raumtemperatur gerührt und anschließend unter vermindertem Druck zur Trockne eingeengt. Durch Umkristallisation des festen Rückstandes aus Diisopropylether wurden Kristalle erhalten (800 mg, 74%).

Schmelzpunkt: 138°C.

DC: R_{f} = 0.30 (Kieselgel; Chloroform-Essigester 60/40 v/v).

IR (CHCl₃): 1731, 1701 (C=O), 1640, 1560, 1538 cm⁻¹ (C=N + C=C).

¹H-NMR (CDCl₃) 300 MHz: 1.26 (t, 3H, CH₃), 2.90 (t, 2H, CH₂), 4.17 (q, 2H, CH₂), 4.34 (t, 2H, CH₂), 7.13 (dd, 1H, arom.), 7.61 (dd, 1H, arom.), 8.71 (m, 1 H, arom.), 10.00 ppm (breites s, 1 H, COOH).

| | | | | |
|---|---|---|---|---|
| CHN-Analyse: | Berechn. | C 51.61; | H 4.69; | N 15.05. |
| | Gefund. | C 51.7; | H 4.7; | N 14.7. |

### 3-[2-(2-Ethoxycarbonyl-1-ethyl)-2,3-dihydro-3-oxo-[1,2,4]triazolo[4,3-a]pyridin-6-carbonylamino]-2-[(phenylmethoxy)carbonylamino]propionsäure-tert-butylester (5.3)

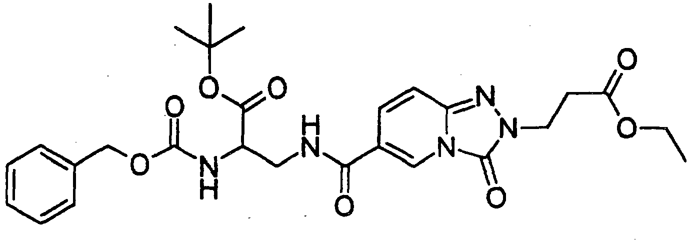

Zu einer Lösung von 2-(2-Ehoxycarbonyl-1-ethyl)-2,3-dihydro-3-oxo-[1,2,4]triazolo[4,3-a]pyridin-6-carbonsäure (800 mg, 2.86 mmol) in trockenem Dimethylformamid (20 ml) wurden nacheinander N,N-Diisopropylethylamin (1.5 ml, 8.6 mmol), O-Benzotriazol-1-yl-N,N,N',N'-tetramethyluronium-tetrafluoroborat (1.25 g, 3.9 mmol) und 3-Amino-2-(benzyloxycarbonylamino)propionsäure-tert-butylester (760 mg, 2.58 mmol) gegeben und die Mischung wurde 6 h bei Raumtemperatur unter Inertgasatmosphäre gerührt. Nach Zugabe von Essigester (100ml) wurde die organische Phase mit 1 N Salzsäure (2x50 ml), gesättigter Natriumhydrogencarbonatlösung (2x50ml) und Kochsalzlösung (1x50 ml) gewaschen, über Magnesiumsulfat getrocknet und unter vermindertem Druck zur Trockne eingeengt. Durch Chromatographie (Kieselgel; Elution mit Essigester/Triethylamin 100/2 v/v) des Rückstandes wurde ein hellgelbes Öl erhalten (800 mg, 50%).

DC: R_{f} = 0.73 (Kieselgel; Essigester/Triethylamin 98/2 v/v).

IR (CHCl₃): 3409, 3344 (NH), 1729, 1670 (C=O), 1641, 1628,1560, 1529, 1507 cm⁻¹ (C=C+ C=N+ Amid).

¹H-NMR (CDCl₃) 300 MHz: 1.24 (t, 3H, CH₃), 1.47 (s, 9H, t-Bu), 2.87 (t, 2H, CH₂), 3.82 (m, 2H, CH₂), 4.15 (q, 2H, CH₂), 4.30 (t, 2H, CH₂), 4.47 (m, 1H, CH), 5.12 (AB, 2H, CH₂Ph), 5.93 (breites d, 1H, NH), 7.06 (dd, 1 H, arom.), 7.20-7.40 (m, 6H, 5arom.+ NH), 7.44 (dd, 1 H, arom.), 8.36 ppm (breites s, 1 H, arom.).

| | | | | |
|---|---|---|---|---|
| CHN-Analyse: | Berechn. | C 58.37; | H 5.99; | N 12.61. |
| | Gefund. | C 58.1; | H 6.0; | N 12.6. |

### 3-[2-(2-Guanidinocarbonyl-1-ethyl)-2,3-dihydro-3-oxo-[1,2,4]triazolo[4,3-a]pyridin-6-carbonylamino]-2-[(phenylmethoxy)carbonylamino]propionsäure-tert-butylester (5.4)

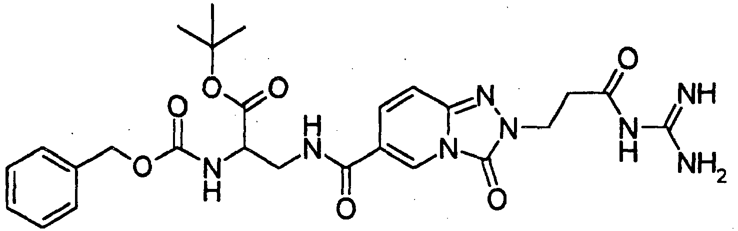

Eine Mischung von 3-[2-(2-Ethoxycarbonyl-1-ethyl)-2,3-dihydro-3-oxo-[1,2,4]triazolo[4,3-a]pyridin-6-carbonylamino]-2-[(phenylmethoxy)carbonylamino]propionsäure-tert-butylester (300 mg, 0.54 mmol) und Guanidin (Base) (160 mg, 2.71 mmol) in trockenem Dimethylformamid (10 ml) wurde 30 h bei Raumtemperatur unter Inertgasatmosphäre gerührt. Die Mischung wurde unter vermindertem Druck zur Trockne eingeengt und der Rückstand chromatographiert (Kieselgel; Elution mit Methylenchlorid/Methanol/Wasser/Essigsäure 90/10/1/1 v/v/v/v). Es wurde ein farbloses Pulver erhalten (90 mg, 29%).

Schmelzpunkt: 120°C Zers.

DC: R_{f} = 0.26 (Kieselgel; Methylenchlorid/Methänol/Wasser/Essigsäure 90/10/1/1 v/v/v/v).

IR (CHCl₃): 3360 (NH/NH₂), 1730, 1714 (C=O), 1670, 1603, 1539, 1531 cm⁻¹ (C=O + C=N + C=C + NH/NH₂).

¹H-NMR (DMSO-d₆) 300 MHz: 1.35 (s, 9H, tBu), 2.61 (t, 2H, CH₂), 3.57 (m, 2H, CH₂), 4.10 (t, 2H, CH₂), 4.22 (q, 1 H, CH), 5.05 (AB, 2H, CH₂Ph), 6.66 (breites m, NH), 7.25-7.40 (m, 5H, arom.), 7.29 (m, 1H, arom.), 7.53 (dd, 1 H, arom.), 7.68 (d, 1 H, NH), 7.70 (breites m, NH), 8.44 (breites s, 1H, arom.), 8.65 ppm (t, 1 H, NH).

Massenspektrum: 569 (MH+).

| | | | | |
|---|---|---|---|---|
| CHN-Analyse: | Berechn. | C 54.92; | H 5.67; | N 19.71. |
| | Gefund. | C 52.5; | H 5.9; | N 17.6. |

### 3-[2-(2-Guanidinocarbonyl-1-ethyl)-2,3-dihydro-3-oxo-[1,2,4]triazolo[4,3-a]pyridin-6-carbonylamino]-2-[(phenylmethoxy)carbonylamino]propionsäure (5.5)

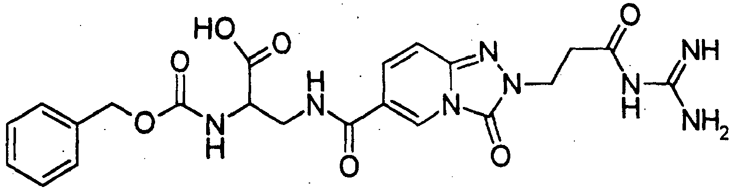

Zu einer auf 0°C gekühlten Lösung von 3-[2-(2-Guanidinocarbonyl-1-ethyl)-2,3-dihydro-3-oxo-[1,2,4]triazolo[4,3-a]pyridin-6-carbonylamino]-2-[(phenylmethoxy)carbonylamino]propionsäure-tert-butylester (85 mg, 0.15 mmol) in Methylenchlorid (10 ml) wurde Trifluoressigsäure (3 ml) gegeben. Nachdem die Mischung auf Raumtemperatur gekommen war, wurde weitere 5 h gerührt, mit Toluol (20 ml) versetzt und die Mischung unter vermindertem Druck zur Trockne eingeengt. Durch zweimalige Chromatographie (Kieselgel; Elution mit Chloroform/Methanol/Wasser/Essigsäure 70/30/6/3 v/v/v/v) des Rückstandes wurde ein blaßgelbes Pulver erhalten (45 mg, 58%).

Schmelzpunkt: 125°C Zers.

DC: R_{f} = 0.39 (Kieselgel; Chtoroform/Methanol/Wasser/Essigsäure 70/30/6/3 v/v/v/v).

IR (Nujol): 3340 (NH/OH), 1706 (C=O), 1653 (C=O + C=N), 1600, 1545, 1530, 1499 cm⁻¹ (arom. + Amid).

¹H-NMR (DMSO-d₆) 300 MHz: 2.63 (t,2H, CH₂), 3.48 (m, 2H, CH₂), 3.92 (m, 1H, CH), 4.09 (t, 2H, CH₂), 4.99 (AB, 2H, CH₂Ph), 6.73 (d, 1 H, NH), 7.00 (breites m, NH), 7.15-7.40 (m, 7H, arom.+ NH), 7.48 (breites d, 1H, arom.), 8.00 (breites m, NH), 8.34 (s, 1H, arom.), 8.70 (sl, NH).

Massenspektrum: 513 (MH+), 535 (MNa+), 551 (MK+).

| | | | | |
|---|---|---|---|---|
| CHN-Analyse: | Berechn. | C 51.56; | H 4.72; | N 21.86. |
| | Gefund. | C 48.7; | H 4.7; | N 18.2. |

### Beispiel 6

### 3-[2-(3-Guanidinocarbonyl-1-propyl)-2,3-dihydro-3-oxo-[1,2,4]triazolo[4,3-a]pyridin-6-carbonylamino]-2-[(phenylmethoxy)carbonylamino]propionsäure (6.5)

Die Synthese wurde nach den folgenden Vorschriften durchgeführt.

### 2-(3-Ethoxycarbonyl-1-propyl)-2,3-dihydro-3-oxo-[1,2,4]triazolo[4,3-a]pyridin-6-carbonsäure-tert-butylester (6.1)

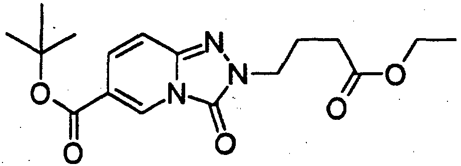

Eine Mischung von 2,3-Dihydro-3-oxo-[1,2,4]triazolo[4,3-a]pyridin-6-carbonsäure-tert-butylester [hergestellt nach WO 94-18981] (1.00 g, 4.25 mmol), Cesiumcarbonat (2.70 g, 8.28 mmol) und 4-Brombuttersäureethylester (1.24 g, 6.36 mmol) in Acetonitril (100 ml) wurde 1 h unter Rückfluß erhitzt und über Nacht bei Raumtemperatur gerührt. Der Niederschlag wurde abfiltriert und das Filtrat wurde unter vermindertem Druck zur Trockne eingeengt. Durch Chromatographie (Kieselgel; Elution mit Methylenchlorid/Essigester 80/20 v/v) des Rückstandes wurde ein hellgelbes Öl erhalten (1.30 g, 87%).

DC: R_{f} = 0.58 (Kieselgel; Chloroform/Essigester 60/40 v/v).

IR (CHCl₃): 1717 (C=O), 1641, 1557, 1536 cm⁻¹ (C=N + C=C).

¹H-NMR (CDCl₃) 250 MHz: 1.25 (t, 3H, CH₃), 1.58 (s, 9H, t-Bu), 2.18 (m, 2H, CH₂), 2.40 (m, 2H, CH₂), 4.07 (t, 2H, CH₂), 4.13 (q, 2H, CH₂), 7.07 (dd, 1 H, arom.), 7.57 (dd, 1 H, arom.), 8.46 ppm (t, 1 H, arom.).

| | | | | |
|---|---|---|---|---|
| CHN-Analyse: | Berechn. | C 58.44; | H 6.64; | N 12.03. |
| | Gefund. | C 58.4; | H 6.7; | N 11.9. |

### 2-(3-Ethoxycarbonyl-1-propyl)-2,3-dihydro-3-oxo-[1,2,4]triazolo[4,3-a]pyridin-6-carbonsäure (6.2)

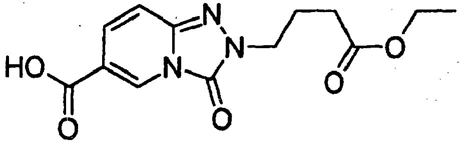

Zu einer auf 0°C gekühlten Lösung von 2-(3-Ethoxycarbonyl-1-propyl)-2,3-dihydro-3-oxo-[1,2,4]triazolo[4,3-a]pyridin-6-carbonsäure-tert-butylester (1.3 g, 3.7 mmol) in Methylenchlorid (20 ml) wurde Trifluoressigsäure (20 ml) gegeben. Die Mischung wurde 1 h bei 0°C und 6 h bei Raumtemperatur gerührt und dann unter vermindertem Druck zur Trockne eingeengt. Durch Umkristallisation des festen Rückstandes aus Diisopropylether wurde Kristalle erhalten (750 mg, 68%).

Schmelzpunkt: 140°C.

DC: R_{f} = 0.16 (Kieselgel; Chloroform/Essigester 60/40 v/v).

IR (CHCl₃): 1728, 1701 (C=O), 1640, 1560, 1535 cm⁻¹ (C=N + C=C).

¹H-NMR (CDCl₃) 300 MHz: 1.25 (t, 3H, CH₃), 2.20 (m, 2H, CH₂), 2.42 (t, 2H, CH₂), 4.10 (m, 2H, CH₂), 4.14 (q, 2H, CH₂), 7.04 (dd, 1 H, arom.), 7.62 (dd, 1H, arom.), 8.72 (t, 1 H, arom.), 8.70 ppm (breites s, 1 H, COOH).

| | | | | |
|---|---|---|---|---|
| CHN-Analyse: | Berechn. | C 53.24; | H 5.16; | N 14.33. |
| | Gefund. | C 53.3; | H 5.1; | N 14.3. |

### 3-[2-(3-Ethoxycarbonyl-1-propyl)-2,3-dihydro-3-oxo-[1,2,4]triazolo[4,3-a]pyridin-6-carbonylamino]-2-[(phenylmethoxy)carbonylamino]propionsäure-tert-butylester (6.3)

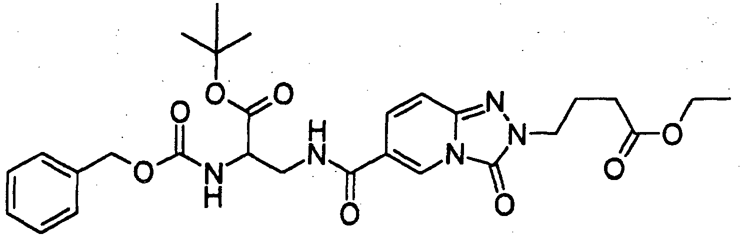

Zu einer Lösung von 2-(3-Ethoxycarbonyl-1-propyl)-2,3-dihydro-3-oxo-[1,2,4]triazolo[4,3-a]pyridin-6-carbonsäure (950 mg, 3.24 mmol) in trockenem Dimethylformamid (20 ml) wurden nacheinander N,N-Diisopropylethylamin (1.69 ml, 9.70 mmol), O-Benzotriazol-1-yl-N,N,N',N'-tetramethyluronium-tetrafluoroborat (1.41 g, 4.40 mmol) und 3-Amino-2-(benzyloxycarbonylamino)propionsäure-tert-butylester (900 mg, 3.06 mmol) gegeben und die Mischung wurde 48 h bei Raumtemperatur unter Inertgasatmosphäre gerührt. Nach Zugabe von Essigester (100ml) wurde die organische Phase mit 1 N Salzsäure (2x50 ml), gesättigter Natriumhydrogencarbonatlösung (2x50ml) und Kochsalzlösung (1x50 ml) gewaschen, über Magnesiumsulfat getrocknet und unter vermindertem Druck zur Trockne eingeengt. Durch Chromatographie (Kieselgel; Elution mit Essigester/Triethylamin 98/2 v/v) des Rückstandes wurde ein hellgelbes Öl erhalten (1.00 g, 54%).

DC: R_{f} = 0.75 (Kieselgel; Essigester/Triethylamin 98/2 v/v).

IR (CHCl₃): 3409, 3344 (NH), 1726, 1670 (C=O), 1641, 1629, 1560, 1529, 1506 cm⁻¹ (C=C + C=N + Amid).

¹H-NMR (CDCl₃) 300 MHz: 1.24 (t, 3H, CH₃), 1.47 (s, 9H, t-Bu), 2.17 (m, 2H, CH₂), 2.39 (t, 2H, CH₂), 3.81 (m, 2H, CH₂), 4.06 (t, 2H, CH₂), 4.12 (q, 2H, CH₂), 4.46 (m, 1H, CH), 5.12 (AB, 2H, CH₂Ph), 5.96 (breites d, 1 H, NH), 7.07 (dd, 1H, arom.), 7.31 (m, 5H, arom.), 7.34 (m, 1 H, NH), 7.44 (dd, 1 H, arom.), 8.36 ppm (breites s, 1 H, arom.).

| | | | | |
|---|---|---|---|---|
| CHN-Analyse: | Berechn. | C 59.04; | H 6.19; | N 12.29. |
| | Gefund. | C 58.7; | H 6.1; | N 12.2. |

### 3-[2-(3-Guanidinocarbonyl-1-propyl)-2,3-dihydro-3-oxo-[1,2,4]triazolo[4,3-a]pyridin-6-carbonylamino]-2-[(phenylmethoxy)carbonylamino]propionsäure-tert-butylester (6.4)

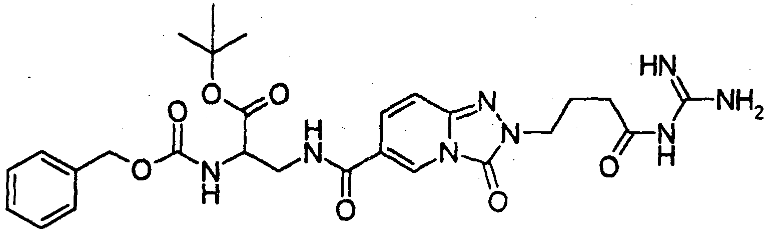

Eine Mischung von 3-[2-(3-Ethoxycarbonyl-1-propyl)-2,3-dihydro-3-oxo-[1,2,4]triazolo[4,3-a]pyridin-6-carbonylamino]-2-[(phenylmethoxy)carbonylamino]propionsäure-tert-butylester (295 mg, 0.52 mmol) und Guanidin (Base) (152 mg, 2.57 mmol) in trockenem Dimethylformamid (10 ml) wurde 30 h bei Raumtemperatur unter Inertgasatmosphäre gerührt. Die Mischung wurde unter vermindertem Druck zur Trockne eingeengt und der Rückstand chromatographiert (Kieselgel; Elution mit Methylenchlorid/Methanol/Wasser/Essigsäure 70/30/1/1 v/v/v/v). Es wurde ein hellgefärbtes Pulver erhalten (60 mg, 20%).

Schmelzpunkt: 110°C Zers.

DC: R_{f} = 0.20 (Kieselgel; Methylenchlorid/Methanol/Wasser/Essigsäure 90/10/1/1 v/v/v/v).

IR (CHCl₃): 3364 (NH/NH₂), 1713 (C=O), 1669, 1602, 1532, cm⁻¹ (C=O + C=N + C=C + NH/NH₂).

¹H-NMR (DMSO-d₆) 300 MHz: 1.35 und 1.39(s, 9H, tBu), 1.95(m, 2H, CH₂), 2.17 (t, 2H, CH₂), 3.57 (m, 2H, CH₂), 3.90 (t, 2H, CH₂), 4.21 (q, 1H, CH), 5.04 (AB, 2H, CH₂Ph), 7.25-7.40 (m, 5H, arom.), 7.27 (d, 1 H, arom.), 7.52 (dd, 1 H, arom.), 7.70 (breites d, NH), 8.45 (breites s, 1 H, arom.), 8.65 ppm (t, 1H, NH).

Massenspektrum: 583 (MH+).

| | | | | |
|---|---|---|---|---|
| CHN-Analyse: | Berechn. | C 55.66; | H 5.88; | N 19.23. |
| | Gefund. | C 51.8; | H 6.1; | N 14.8. |

### 3-[2-(3-Guanidinocarbonyl-1-propyl)-2,3-dihydro-3-oxo-[1,2,4]triazolo[4,3-a]pyridin-6-carbonylamino]-2-[(phenylmethoxy)carbonylamino]propionsäure (6.5)

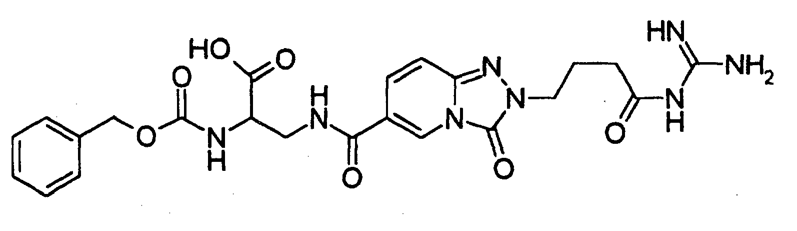

Zu einer auf 0°C gekühlten Lösung von 3-[2-(3-Guanidinocarbonyl-1-propyl)-2,3-dihydro-3-oxo-[1,2,4]triazolo[4,3-a]pyridin-6-carbonylamino]-2-[(phenylmethoxy)carbonylamino]propionsäure-tert-butylester (60 mg, 0.10 mmol) in Methylenchlorid (10 ml) wurde Trifluoressigsäure (3 ml) gegeben. Nachdem die Mischung auf Raumtemperatur gekommen war, wurde weitere 7 h gerührt, mit Toluol (20 ml) versetzt und unter vermindertem Druck zur Trockne eingeengt. Durch Chromatographie (Kieselgel; Elution mit Chloroform/Methanol/Wasser/Essigsäure 70/30/6/3 v/v/v/v) des Rückstandes wurde ein schwachgelbes Pulver erhalten (17 mg, 31%).

Schmelzpunkt: 190°C Zers.

DC: R_{f} = 0.50 (Kieselgel; Chloroform/Methanol/Wasser/Essigsäure 70/30/6/3 v/v/v/v).

IR (Nujol): 1705 (C=O), 1650 (C=O + C=N), 1595, 1525 cm⁻¹ (arom. + Amid).

¹H-NMR (DMSO-d₆) 300 MHz: 1.98 (m, 2H, CH₂), 2.34 (t, 2H, CH₂), 2.50 (hidden m, 2H, CH₂), 3.89 (t, 2H, CH₂), 3.95 (q, 1 H, CH), 4.99 (m, 2H, CH₂Ph), 6.81 (breites s, 1H, NH), 7.20-7.38 (m, 7H, arom.+ NH), 7.47 (d, 1 H, arom.), 8.30 (breites s, 1 H, NH), 8.36 (s, 1 H, arom.), 8.44 (breites s, NH).

Massenspektrum: 527 (MH+), 549 (MNa+).

### Beispiel 7

### 2-Benzyloxycarbonylamino-3-[6-(guanidinocarbonylmethyloxy)-3,4-dihydro-1-oxo-2(1H)-isochinolyl]propionsäure (7.10)

Die Synthese wurde nach den folgenden Vorschriften durchgeführt.

### 2-(Acetylamino)-3-[6-(benzyloxy)-3,4-dihydro-1-oxo-2(1H)-isochinolyl]propionsäuremethylester (7.1)

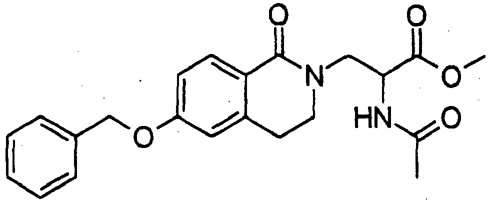

Zu einer Suspension von 6-(Benzyloxy)-3,4-dihydro-1-oxo-2(1H)-isochinolin [hergestellt nach der in J.Med.Chem. (1997), 40(13), 2085-2101 beschriebenen Methode] (1.41g, 5.57 mmol) und von trockenem Cesiumfluorid (1.68 g, 11.06 mmol) in Tetrahydrofuran (15ml) wurde unter Inertgasatmosphäre bei Raumtemperatur Tetramethoxysilan (4 ml, 27.12 mmol) gegeben. Die Mischung wurde 1 h bei Raumtemperatur gerührt und dann auf 50°C erhitzt. Bei dieser Temperatur wurde innerhalb 1 h eine Lösung von 2-Acetamidoacrylsäuremethylester (1.60 g, 11.18 mmol) in Tetrahydrofuran (7 ml) zugegeben. Die Mischung wurde 1 h bei 50°C gehalten und nach dem Abkühlen auf Raumtemperatur unter vermindertem Druck zur Trockne eingeengt. Durch Chromatographie ( Kieselgel; Elution mit Essigester/Methylenchlorid 50/50 v/v) des Rückstandes wurde ein Öl erhalten (0.7 g, 31 %).

DC: R_{f} = 0.26 ( Essigester/Methylenchlorid 60/40 v/v).

¹H-NMR (CDCl₃) 250 MHz: 1.95 (s, 3H, CH₃), 2.80-3.00 (m, 2H, CH₂), 3.40-3.65 (m, 2H, CH₂), 3.65-3.80 (m, 1H,CH₂), 3.70 (s, 3H, CH₃), 3.90-4.05 (m, 1 H, CH₂), 4.60-4.80 (m, 1H, CH), 5.05 (s, 2H, CH₂Ph), 6.70 (d, 1H, arom.), 6.90 (dd, 1 H, arom.), 7.05 (d, 1H, NH), 7.25-7.45 (m, 5H, arom.), 7.95 ppm (d, 1H, arom.).

### 2-Amino-3-[6-(benzyloxy)-3,4-dihydro-1-oxo-2(1H)-isochinolyl]propionsäuremethylester (7.2)

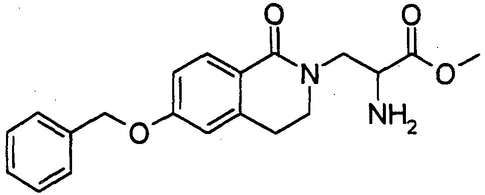

Eine Mischung von 2-(Acetylamino)-3-[6-(benzyloxy)-3,4-dihydro-1-oxo-2(1H)-isochinolyl]propionsäuremethylester (0.97 g, 2.45 mmol) in Methanol (25 ml) und von konzentrierter Salzsäure (4.2 ml) wurde 92 h bei 50°C gerührt. Nach dem Abkühlen auf Raumtemperatur wurde die Mischung unter vermindertem Druck zur Trockne eingeengt und der Rückstand chromatographiert (Kieselgel; Elution mit Methanol/Methylenchlorid 3/97 v/v). Es wurden Kristalle erhalten (0.19 g, 21 %).

Schmelzpunkt: 104°C

DC: R_{f} = 0.05 (Kieselgel; Methanol/Methylenchlorid 5/95 v/v).

IR (CHCl₃): 3390, 3316 (NH₂), 1737, 1643 (C=O), 1606, 1578, 1498 cm⁻¹ (arom. + NH₂).

¹H-NMR (CDCl₃) 250 MHz: 1.67 (breites s, 2H, NH₂), 2.85-3.05 (m, 2H, CH₂), 3.50-3.95 (m, 5H, 2CH₂ + CH), 3.75 (s, 3H, CH₃), 5.20 (s, 2H, CH₂), 6.75 (d, 1 H, arom.), 6.91 (dd, 1 H, arom.), 7.25- 7.45 (m, 5H, arom.), 8.00 ppm (d, 1H, arom.).

Massenspektrum: 355 (MH+), 377 (MNa+).

| | | | | |
|---|---|---|---|---|
| CHN-Analyse: | Berechn. | C 67.78; | H 6.26; | N 7.90. |
| | Gefund. | C 67.3; | H 6.4; | N 7.9. |

### 2-Benzyloxycarbonylamino-3-[6-(benzyloxy)-3,4-dihydro-1-oxo-2(1H)-isochinolyl]propionsäuremethylester (7.3)

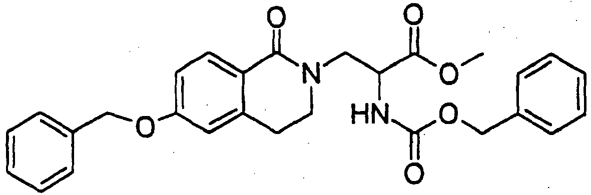

Zu einer Suspension von 2-Amino-3-[6-(benzyloxy)-3,4-dihydro-1-oxo-2(1H)-isochinolyl]propionsäuremethylester (0.61 g, 1.72 mmol) in 1,2-Dimethoxyethan (6 ml) wurde bei Raumtemperatur und unter Inertgasatmosphäre eine Lösung von N-(Benzyloxycarbonyloxy)succinimid (0.45 g, 1.80 mmol) in 1,2-Dimethoxyethan (13 ml) gegeben. Die Mischung wurde 2 h bei Raumtemperatur gerührt und dann unter vermindertem Druck zur Trockne eingeengt. Durch Chromatographie ( Kieselgel; Elution mit Methanol/Methylenchlorid 3/97 v/v) des Rückstandes wurde ein Öl erhalten (0.84 g, quantitativ).

DC: R_{f} = 0.25 (Kieselgel; Methanol/Methylenchlorid 5/95 v/v).

IR (CHCl₃): 3422 (NH), 1746, 1719, 1642 (C=O), 1606, 1578, 1512, 1500, 1480 cm⁻¹ (arom. + Amid).

¹H-NMR (CDCl₃) 300 MHz: 2.81 (t, 2H, CH₂), 3.45-3.60 (m, 2H, CH₂), 3.76 (s, 3H, CH₃), 3.80 (dd, 1 H, CH₂), 4.03 (dd, 1H, CH₂), 4.45-4.57 (m, 1 H, CH), 5.06 (m, 4H, CH₂Ph), 6.14 (breites d, 1H, NH), 6.73 (d, 1H, arom.), 6.92 (dd, 1 H, arom.), 7.28 (m, 5H, arom.), 7.40 (m, 5H, arom.), 7.97 ppm (d, 1 H, arom.).

Massenspektrum: 489 (MH+), 511 (MNa+).

### 2-Benzyloxycarbonylamino-3-[6-(benzyloxy)-3,4-dihydro-1-oxo-2(1 H)-isochinolyl]propionsäure (7.4)

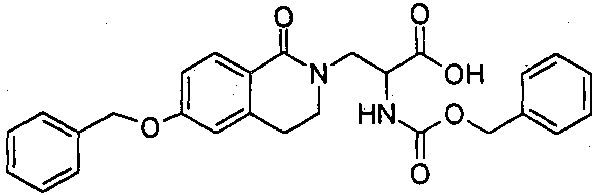

Zu einer Lösung von 2-Benzyloxycarbonylamino-3-[6-(benzyloxy)-3,4-dihydro-1-oxo-2(1H)-isochinolyl] propionsäuremethylester (0.84 g, 1.72 mmol) in Methanol (6 ml) wurde bei Raumtemperatur eine Lösung von 2N NaOH (1.2 ml, 2.4 mmol) gegeben und die Mischung 2 h bei dieser Temperatur gerührt. Nach Einengen zur Trockne unter vermindertem Druck wurde der Rückstand chromatographiert (Kieselgel; Elution mit Methylenchlorid/Methanol/Essigsäure 98/2/1 v/v/v). Es wurde ein Öl erhalten (0.73 g, 89%).

DC: R_{f} = 0.36 (Kieselgel; Methanol/Methylenchlorid 10/90 v/v).

IR (CHCl₃): 3412 (NH), 1746, 1714, 1637 (C=O), 1604, 1577, 1507, 1498 cm⁻¹ (arom. + Amid).

¹H-NMR (CDCl₃) 250 MHz: 2.85 (m, 2H, CH₂), 3.45-4.50 (m, 5H, 2CH₂ + CH), 5.06 (breites s, 4H, 2CH₂Ph), 6.68 (breites s, 1 H, arom.), 6.85 (m, 1 H, arom.), 7.20-7.40 (m, 10H, arom.), 7.94 ppm (m, 1 H, arom.).

Massenspektrum: 473 (M-H).

### 2-Benzyloxycarbonylamino-3-[6-(benzyloxy)-3,4-dihydro-1-oxo-2(1H)-isochinolyl]propionsäure-tert-butylester (7.5)

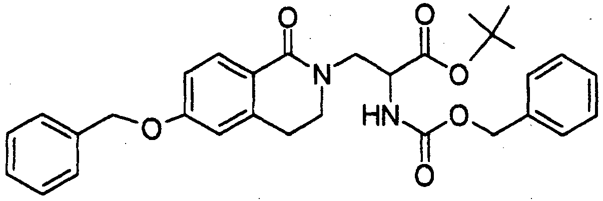

Eine Mischung von 2-Benzyloxycarbonylamino-3-[6-(benzyloxy)-3,4-dihydro-1-oxo-2(1 H)-isochinolyl]propionsäure (0.71 g, 1.49 mmol) und Dimethylformamid-di-tert-butylacetal (15 ml) wurde 3 h unter Inertgasatmosphäre auf 90°C erhitzt. Die Lösung wurde unter vermindertem Druck zur Trockne eingeengt und der Rückstand chromatographiert (Kieselgel; Elution mit Methanol/Methylenchlorid 1/99 v/v). Es wurde ein Öl erhalten (0.57 g, 72%).

DC: R_{f} = 0.73 (Kieselgel; Methanol/Methylenchlorid 10/90 v/v).

IR (CHCl₃): 3424 (NH), 1740, 1714, 1648, 1631 (C=O), 1606, 1579, 1505, 1499 cm⁻¹ (arom. + Amid).

¹H-NMR (CDCl₃) 250 MHz: 1.35 (s, 9H, tBu), 2.80-3.10 (m, 2H, CH₂), 3.50-3.75 (m, 2H, CH₂), 3.85 (mAB, 2H, CH₂), 4.45 (m, 1H, CH), 5.05 (s, 2H, CH₂Ph), 5.10 (s, 2H, CH₂Ph), 5.90 (breites d, 1H, NH), 6.65 (d, 1 H, arom.), 6.90 (dd, 1 H, arom.), 7.10-7.40 (m, 10H, arom.), 7.90 ppm (d, 1 H, arom.).

Massenspektrum: 553 (MNa+).

### 2-Amino-3-[3,4-dihydro-6-hydroxy-1-oxo-2(1H)-isochinolyl]propionsäure tert-butyl ester (7.6)

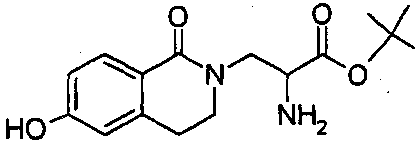

Eine Mischung von 2-Benzyloxycarbonylamino-3-[6-(benzyloxy)-3,4-dihydro-1-oxo-2(1 H)-isochinolyl]propionsäure-tert-butylester (0.55 g, 1.03 mmol), Palladiumhydroxid (20 %ig auf Aktivkohle) (0.28 g) und Cyclohexen (2.4 ml) in Methanol (15 ml) wurde 3 h unter Rückfluß erhitzt. Es wurde über Clarcel filtriert und das Fitlrat unter vermindertem Druck zur Trockne eingeengt. Durch Chromatographie (Kieselgel; Elution mit Methanol/Methylenchlorid 5/95 v/v) des Rückstandes wurde ein Öl erhalten (0.11 g, 34%).

DC: R_{f} = 0.15 (Kieselgel; Methylenchlorid/Methanol/Essigsäure/Wasser 90/10/1/1 v/v/v/v).

¹H-NMR (CDCl₃) 250 MHz: 1.40 (s, 9H, t-Bu), 2.65-2.80 (m, 2H, CH₂), 3.45-3.75 (m, 4H, CH₂ + austauschbar), 3.80-4.20 (m, 4H, CH + CH₂ + austauschbar), 6.35 (breites s , 1H, arom.), 6.55 (dd, 1H, arom.), 7.65 ppm (d, 1H, arom.).

Massenspektrum: 305 (M-H), 249 (M-tBu),162.

### 2-Benzyloxycarbonylamino-3-[3,4-dihydro-6-hydroxy-1-oxo-2(1H)-isochinolyl]propionsäure tert-butyl ester (7.7)

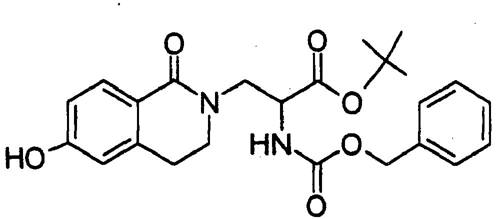

Zu einer Lösung von 2-Amino-3-[3,4-dihydro-6-hydroxy-1-oxo-2(1H)-isochinolyl]propionsäure-tert-butylester (0.110 g, 0.36 mmol) in 1,2-Dimethoxyethan (1.3 ml) wurde tropfenweise bei Raumtemperatur und unter Inertgasatmosphäre eine Lösung von N-(Benzyloxycarbonyloxy)succinimid (0.094 g, 0.38 mmol) in 1,2-Dimethoxyethan (3 ml) gegeben. Die Mischung wurde 2 h bei Raumtemperatur gerührt, unter vermindertem Druck zur Trockne eingeengt und der Rückstand chromatographiert (Kieselgel; Elution mit Methylenchlorid/Methanol 97/3 v/v). Es wurde ein Öl erhalten (0.16 g, quantitativ).

DC: R_{f} = 0.50 (Kieselgel; Methylenchlorid/Methanol 90/10 v/v).

IR (CHCl₃): 3594 (OH), 3424 (NH), 1744, 1715, 1642 (C=O), 1611, 1587, 1507, 1480 cm⁻¹ (arom.+ Amid).

¹H-NMR (CDCl 3) 250 MHz: 1.43 (s, 9H, t-Bu), 2.85 (m, 2H, CH₂), 3.58 (m, 2H, CH₂), 3.78 und 3.99 (AB dd, 2H, CH₂), 4.47 (m, 1 H, CH), 5.06 (s, 2H, CH₂Ph), 5.84 (breites d, 1 H, NH), 6.59 (d, 1H, arom.), 6.75 (dd, 1 H, arom.), 7.27 (breites s, 5H, arom.), 7.90 (d, 1 H, arom.).

Massenspektrum: 439 (M-H), 275, 162.

### 2-Benzyloxycarbonylamino-3-[3,4-dihydro-6-(methoxycarbonylmethyloxy)-1-oxo-2(1 H)-isochinolyl]propionsäure tert-butyl ester (7.8)

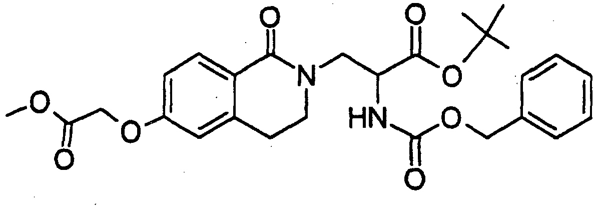

Eine Mischung von 2-Benzyloxycarbonylamino-3-[3,4-dihydro-6-hydroxy-1-oxo-2(1 H)-isochinolyl]propionsäure-tert-butylester (140 mg, 0.32 mmol), Cesiumcarbonat (220 mg, 0.67 mmol) und 2-Bromessigsäuremethylester (56 mg, 0.37 mmol) in Acetonitril (10 ml) wurde 1/2 h unter Rückfluß erhitzt. Die Mischung wurde unter vermindertem Druck zur Trockne eingeengt und der Rückstand chromatographiert (Kieselgel; Elution mit Methylenchlorid/Essigester 90/10 v/v). Es wurde ein Öl erhalten (117 mg, 72%).

DC: R_{f} = 0.45 (Kieselgel; Methylenchlorid/Essigester 80/20 v/v).

IR (CHCl₃): 3420 (NH), 1754, 1737, 1717, 1644 (C=O), 1607, 1580, 1513, 1502 (arom.+ Amid), 1440 (CO₂Me), 1370 cm⁻¹ (CO₂tBu).

¹H-NMR (CDCl₃) 250 MHz: 1.43 (s, 9H, tBu), 2.90 (t, 2H, CH₂), 3.59 (m, 2H, CH₂), 3.82 (s, 3H, CH₃), 3.90- 4.20 (m, 2H, CH₂), 4.47 (m, 1 H, CH), 4.68 (s, 2H, CH₂CO), 5.04 (s, 2H, CH₂Ph), 5.89 (d, 1 H, NH), 6.67 (d, 1 H, arom.), 6.82 (dd, 1H, arom.), 7.24-7.40 (m, 5H, arom.), 8.00 ppm (d, 1 H, arom.).

Massenspektrum: 535 (MNa+).

### 2-Benzyloxycarbonylamino-3-[6-(guanidinocarbonylmethyloxy)-3,4-dihydro-1-oxo-2(1H)-isochinolyl]propionsäure-tert-butylester (7.9)

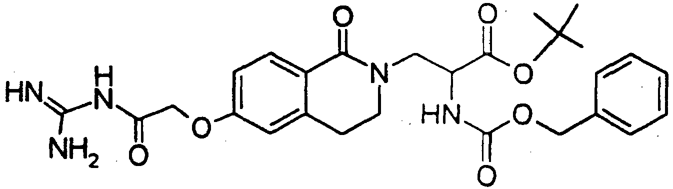

Eine Mischung von 2-Benzyloxycarbonylamino-3-[3,4-dihydro-6-(methoxycarbonylmethyloxy)-1-oxo-2(1H)-isochinolyl]propionsäure-tert-butylester (55 mg, 0.11 mmol) und Guanidin (Base) (13 mg, 0.22 mmol) in trockenem Dimethylformamid (1 ml) wurde 1 h bei Raumtemperatur unter Inertgasatmosphäre gerührt. Die Mischung wurde unter vermindertem Druck zur Trockne eingeengt, der Rückstand in der gerade erforderlichen Menge Methylenchlorid/Methanol gelöst und die Lösung in Diisopropylether gegossen. Der ausgefallene Festastoff wurde abfiltriert und im Vakuum getrocknet. Es wurde ein amorphes Pulver erhalten (18 mg, 31%).

DC: R_{f} = 0.60 (Kieselgel; Methylenchlorid/Methanol/Essigsäure/Wasser 85/15/2/2 v/v/v/v).

IR (CHCl₃): 3508, 3475, 3418 (NH/NH₂), 1736, 1716 (C=O), 1639 (C=O), 1606, 1577, 1516, 1500, 1480 cm⁻¹ (C=N+ arom.+ Amid+ NH/NH₂).

¹H-NMR (DMSO-d₆) 300 MHz: 1.26-1.34 (2s, 9H, tBu), 2.63 (m, 2H, CH₂), 3.31 (m, 1 H, CH₂), 3.53 (m, 2H, CH₂), 4.11 (dd, 1 H, CH₂), 4.29 (m, 1H, CH), 4.49 (s, 2H, CH₂CO), 5.02 (s, 2H, CH₂Ph), 6.69 (d,1H, arom.), 6.78 (dd, 1H, arom.), 7.26-7.31 (m, 5H, arom.), 7.74 ppm (d, 1 H, arom.).

Massenspektrum: 562 (MNa+), 540 (MH+), 484 (M-tBu), 440, 423.

| | | | | |
|---|---|---|---|---|
| CHN-Analyse: | Berechn. | C 60.10; | H 6.16; | N 12.98. |
| | Gefund. | C 58.5; | H 6.1; | N 12.8. |

### 2-Benzyloxycarbonylamino-3-[6-(guanidinocarbonylmethyloxy)-3,4-dihydro-1-oxo-2(1H)-isochinolyl]propionsäure (7.10)

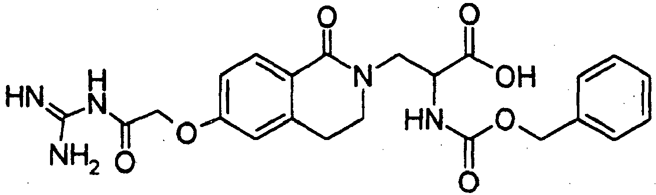

Zu einer Lösung von 2-Benzyloxycarbonylamino-3-[6-(guanidinocarbonylmethyloxy)-3,4-dihydro-1-oxo-2(1H)-isochinolyl]propionsäure-tert-butylester (37.8 mg, 0.07 mmol) in Methylenchlorid (10 ml) wurde tropfenweise bei Raumtemperatur Trifluoressigsäure (3.5 ml) gegeben. Die Mischung wurde 3 h bei Raumtemperatur gerührt, mit Toluol (20 ml) versetzt und unter vermindertem Druck zur Trockne eingeengt. Der Rückstand wurde in Essigsäure gelöst und mit Diisopropylether gefällt. Nach dem Trocknen des erhaltenen Pulvers im Vakuum wurde ein amorpher Feststoff erhalten (8.7 mg, 25%).

DC: R_{f} = 0.25 (Kieselgel; Methylenchlorid/Methanol/Essigsäure/Wasser 85/15/2/2 v/v/v/v).

IR (Nujol): 1704,1634 (C=O), 1602 cm⁻¹ (arom.+ Amid).

¹H-NMR (DMSO-d₆) 300 MHz: 2.79 (m, 2H, CH₂), 3.52 (m, 2H, CH₂), 3.52 und 3.85 (2m, 2H, CH₂), 4.11 (m, 1 H, CH), 4.49-4.86 (2s, 2H, CH₂CO), 4.95 (AB, 2H, CH₂Ph), 6.66 und 6.70 (2d, 1 H, arom.), 6.77 und 6.87 (2dd, 1H, arom.), 7.25-7.31 (m, 5H, arom.), 7.74 und 7.78 ppm (2d, 1 H, arom.).

Massenspektrum: 506 (MNa+), 484 (MH+).

Die Hemmung der Knochenresorption durch die erfindungsgemäßen Verbindungen kann beispielsweise mit Hilfe eines Osteoclasten-Resorptions-Tests ("PIT ASSAY") beispielsweise analog WO 95/32710 bestimmt werden. Die Testmethoden, nach denen die antagonistische Wirkung der erfindungsgemäßen Verbindungen auf den Vitronectinrezeptor αᵥß₃ bestimmt werden können, sind nachfolgend beschrieben.

### Testmethode 1:

Inhibierung der Bindung von humanem Vitronectin (Vn) an humanen Vitronectinrezeptor (VnR) αᵥß₃: ELISA-Test.

### (Testmethode 1 wird abgekürzt mit Vn/VnR bezeichnet)

### 1. Reinigung von humanem Vitronectin

Humanes Vitronectin wird aus menschlichem Plasma isoliert und durch Affinitätschromatographie nach der Methode von Yatohyo et al., Cell Structure and Function, 1988, 23, 281-292 gereinigt.

### 2. Reinigung von humanem Vitronectinrezeptor (αᵥβ₃)

Humaner Vitronectinrezeptor wird aus der menschlichen Plazenta nach der Methode von Pytela et al., Methods Enzymol. 1987, 144, 475 gewonnen. Humaner Vitronectinrezeptor αᵥβ₃ kann auch aus einigen Zellinien (z.B. aus 293 Zellen, einer humanen embryonalen Nierenzellinie), die mit DNA Sequenzen für beide Untereinheiten αᵥ und β₃ des Vitronectinrezeptors cotransfiziert sind, gewonnen werden. Die Untereinheiten werden mit Octylglycosid extrahiert und anschließend über Concanavalin A, Heparin-Sepharose und S-300 chromatographiert.

### 3. Monoklonale Antikörper

Murine monoklonale Antikörper, spezifisch für die ß₃ Untereinheit des Vitronectinrezeptors, werden nach der Methode von Newman et al., Blood, 1985, 227-232, oder nach einem ähnlichen Verfahren hergestellt. Das Kaninchen Fab 2 anti-Maus Fc Konjugat an Meerrettich Peroxidase (anti-Maus Fc HRP) wurde von Pel Freeze (Katalog Nr. 715 305-1) bezogen.

### 4. ELISA Test

Nunc Maxisorp 96 well-Mikrotiter-Platten werden mit einer Lösung von humanem Vitronectin (0.002 mg/ml, 0.05 ml/well) in PBS (Phosphat-gepufferte Kochsalzlösung) über Nacht bei 4°C belegt. Die Platten werden zweimal mit PBS/0.05 % Tween 20 gewaschen und durch Inkubieren (60 min) mit Rinderserum-Albumin (BSA, 0.5 %, RIA Güteklasse oder besser) in Tris-HCl (50 mM), NaCl (100 mM), MgCl₂ (1 mM), CaCl₂ (1 mM), MnCl₂ (1 mM), pH 7, geblockt. Man stellt Lösungen von bekannten Inhibitoren und von den Testsubstanzen in Konzentration von 2x10⁻¹² bis 2x10⁻⁶ Mol/l in Assay-Puffer [BSA (0.5 %, RIA-Güteklasse oder besser) in Tris-HCl (50 mM), NaCl (100 mM), MgCl₂ (1 mM), CaCl₂ (1 mM), MnCl₂ (1 mM), pH 7] her. Die geblockten Platten werden entleert, und jeweils 0.025 ml dieser Lösung, die eine definierte Konzentration (2x10⁻¹² bis 2x10⁻⁶) entweder an einem bekannten Inhibitor oder an einer Testsubstanz enthält, werden in jedes well gegeben..0.025 ml einer Lösung des Vitronectinrezeptors im Testpuffer (0.03 mg/ml) werden in jedes weil der Platte pipettiert und die Platte wird auf einem Schüttler 60-180 min bei Raumtemperatur inkubiert. In der Zwischenzeit wird eine Lösung (6 ml/Platte) eines für die β₃-Untereinheit des Vitronectinrezeptors spezifischen murinen monoklonalen Antikörpers im Assay-Puffer (0.0015 mg/ml) hergestellt. Zu dieser Lösung gibt man einen zweiten Kaninchen-Antikörper (0.001 ml Stammlösung/6 ml der murinen monoklonalen anti-β₃-Antikörper Lösung), der ein anti-Maus-Fc HRP-Antikörper-Konjugat darstellt, und dieses Gemisch aus murinem anti-β₃ Antikörper und Kaninchen anti-Maus Fc HRP Antikörper Konjugat läßt man während der Zeit der Rezeptor-Inhibitor-Inkubation inkubieren.

Die Testplatten werden 4 mal mit PBS-Lösung, die 0.05 % Tween-20 enthält, gewaschen und man pipettiert jeweils 0.05 ml/well der Antikörpermischung in jedes well der Platte und inkubiert 60-180 min.. Die Platte wird 4 mal mit PBS/0.05 % Tween-20 gewaschen und anschließend mit 0.05 ml/well einer PBS-Lösung, die 0.67 mg/ml o-Phenylendiamin und 0.012 % H₂O₂ enthält. entwickelt. Alternativ hierzu kann o-Phenylendiamin in einem Puffer (pH 5) eingesetzt werden. der Na₃PO₄ (50 mM) und Zitronensäure enthält. Die Farbentwicklung wird mit 1 N H₂SO₄ (0,05 ml/well) gestoppt.Die Absorption jedes well wird bei 492-405 nm gemessen und die Daten werden nach Standardmethoden ausgewertet.

### Testmethode 2:

Inhibierung der Bindung von Kistrin an humanen Vitronectinrezeptor (VnR) αᵥβ₃: ELISA-Test

### (Testmethode 2 wird abgekürzt mit KistrinNnR bezeichnet)

### 1. Reinigung von Kistrin

Kistrin wird nach den Methoden von Dennis et al., wie in Proc. Natl. Acad. Sci. USA 1989, 87, 2471-2475 und PROTEINS: Structure, Function and Genetics 1993, 15, 312-321, beschrieben, gereinigt.

### 2. Reinigung von humanem Vitronectinrezeptor (αᵥβ₃)

siehe Testmethode 1.

### 3. Monoklonale Antikörper

siehe Testmethode 1.

### 4. ELISA-Test

Die Fähigkeit von Substanzen, die Bindung von Kistrin an den Vitronectinrezeptor zu inhibieren, kann mit einem ELISA-Test ermittelt werden. Zu diesem Zweck werden Nunc 96 well-Mikrotiterplatten mit einer Lösung von Kistrin (0.002 mg/ml) nach der Methode von Dennis et al., wie in PROTEINS: Structure, Function and Genetics 1993, 15, 312-321, beschrieben, belegt. Die weitere experimentelle Durchführung des ELISA-Test erfolgt wie bei Testmethode 1, Punkt 4, beschrieben.

Testmethode 3:

Inhibierung der Bindung von mit αᵥß₃ tranfizierten 293 Zellen an humanes Vitronectin:

### (Testmethode 3 wird abgekürzt mit Vn/293 Zelltest bezeichnet)

### Zelltest

293 Zellen, eine humane embryonale Nierenzellinie, die mit DNA-Sequenzen für die αᵥ und ß₃ Untereinheiten des Vitronectinrezeptors αᵥß₃ cotransfiziert sind, werden nach der FACS Methode unter dem Gesichtspunkt einer hohen Expressionsrate (> 500000 αᵥß₃ Rezeptoren/Zelle) selektiert. Die ausgewählten Zellen werden kultiviert und erneut mittels FACS aussortiert, um eine stabile Zellinie (15 D) mit Expressionsraten > 1000000 Kopien an αᵥß₃ pro Zelle zu erhalten.

Eine Limbro 96 well-Gewebekulturplatte mit flachem Boden wird mit humanem Vitronectin (0.01 mg/ml, 0.05 ml/well) in Phosphat-gepufferter Kochsalzlösung (PBS) über Nacht bei 4°C belegt und anschließend mit 0.5 %igem BSA geblockt. Man stellt Lösungen der Testsubstanzen von 10⁻¹⁰ bis 2 x 10⁻³ Mol/l in glucosehaltigem DMEM Medium her und gibt jeweils 0.05 ml/well der Lösung auf die Platte. Die Zellen, die hohe Level an αᵥß₃ exprimieren (z.B. 15 D), werden in glucosehaltigem DMEM Medium suspendiert und die Suspension wird auf einem Gehalt von 25000 Zellen/0.05 ml Medium eingestellt. 0.05 ml dieser Zellsuspension werden in jedes weil gegeben und die Platte bei 37°C 90 min inkubiert. Die Platte wird 3 x mit warmen PBS gewaschen um nichtgebundene Zellen zu entfernen. Die gebundenen Zellen werden in Zitratpuffer (25 mM, pH 5.0), der 0.25 % Triton X-100 enthält, lysiert. Anschließend wird das Hexoseamidase-Substrat p-Nitrophenyl-N-acetyl-β-D-glucosaminid zugegeben und die Platte 90 min bei 37°C inkubiert. Die Reaktion wird mit einem Glycin (50 mM)/EDTA (5 mM)-Puffer (pH 10.4) gestoppt und die Absorption von jedem well bei 405-650 nm gemessen.

Die antagonistische Wirkung der erfindungsgemäßen Verbindungen auf den Fibrinogenrezeptor α_{III}β₃, insbesondere zur Selektivitätsbestimmung, kann wie in US 5 403 836, S. 237 beschrieben, durchgeführt werden.

## Patentansprüche

1. Verbindung der Formel I,
A-B-D-E-F-G (I)
worin bedeuten:
A = A₁, mit
A₁ = R²R³N-C(=NR²)NR²C(O)-
B (C₁-C₄)-Alkandiyl,
D eine direkte Bindung, (C₁-C₄)-Alkandiyl,
E 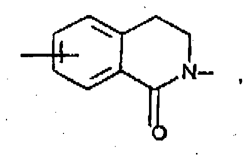 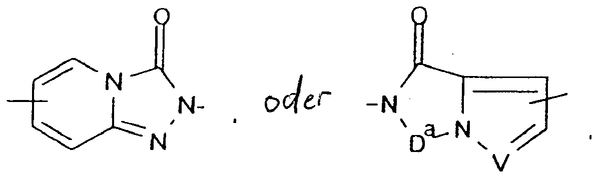
wobei, V N ist und D^{a} CH₂-CH₂ ist
oder 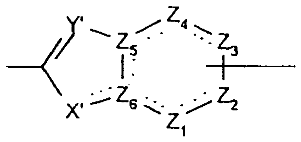 wobei bedeuten:
X' eine -NR^{2b} Grupp
R^{2b} ein Wasserstoffatom ist
Y', ein Stickstoffatom
Z₁, Z₂, Z₃ und Z₄, die gleich oder verschieden sein können, Methingruppen, Kohlenstoffatome, wobei einer der Reste Z₁ bis Z₄ ein Kohlenstoffatom enthalten muß und die drei anderen der Reste Z₁ bis Z₄ Methingruppen sind
Z₅ und Z₆ jeweils ein Kohlenstoffatom
F eine direkte Bindung, (C₁-C₆)-Alkandiyl, -CO-NR²-, die jeweils ein- oder zweifach durch (C₁-C₄)-Alkyl substituiert sein können;
G 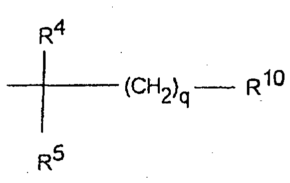
R², R³ unabhängig voneinander H, (C₁-C₄)-Alkyl,
R⁴ R¹⁶OC(O)NH-, R¹⁶C(O)NH-
R⁵ H, (C₁-C₆)-Alkyl, (C₅-C₆)-Cycloalkyl, (C₅-C₆)-Cycloalkyl-(C₁-C₄)-alkyl, Trifluormethyl, Pentafluorethyl, Phenyl, Benzyl;
R⁸ H, (C₁-C₄)-Alkyl, (C₅-C₆)-Cycloalkyl, (C₅-C₆)-Cycloalkyl-(C₁-C₂)-alkyl, Phenyl, Benzyl, Trifluormethyl, Pentafluorethyl;
R¹⁰ C(O)R¹¹;
R¹¹ OH, (C₁-C₆)-Alkoxy, Phenoxy, Benzyloxy, (C₁-C₄)-Alkylcarbonyloxy-(C₁-C₄)-alkoxy, NH₂, Mono-oder Di-(C₁-C₆-alkyl)-amino;
R¹⁶ (C₁₀-C₁₄)-Cycloalkyl, (C₁₀-C₁₄)-Cycloalkyl-(C₁-C₄)-alkyl, die gegebenenfalls 1 oder 2-fach durch (C₁-C₄)-Alkyl, Trifluormethyl, Phenyl, Benzyl, (C₁-C₄)-Alkoxy, Phenoxy, Benzyloxy, =O oder Mono- oder Di-(C₁-C₄-alkyl)-amino substituiert sein können, wobei die Cycloalkylreste bevorzugt 1-Adamantyl oder 2-Adamantyl sind, die wie vorstehend beschrieben, substituiert sein können;
n 1 oder 2; und
q 0
in allen ihren stereoisomeren Formen und Mischungen davon in allen Verhältnissen, und ihren physiologisch verträglichen Salze.

2. Verbindung der Formel I gemäß Ansprüch 1, in denen der Abstand zwischen R¹⁰ und dem ersten N-Atom in A₁ entlang des kürzesten Weges zwischen diesen Atomen 12 bis 13, kovalente Bindungen beträgt, in allen ihren stereoisomeren Formen und Mischungen davon in allen Verhältnissen, und ihre physiologisch verträglichen Salze.

3. Verbindung gemäß Ansprüch 1, (2S)-3-[5-(2-Guanidinocarbonyl-ethyl)-4,5,6,7-tetrahydro-4-oxo-pyrazolo[1,5-a]-pyrazin-2-carbonylamino]-2-[benzyloxycarbonylamino]-propionsäure

4. Verbindung gemäß Ansprüch 1, (2S)-3-[5-(3-Guanidinocarbonyl-propyl)-4,5,6,7-tetrahydro-4-oxo-pyrazolo[1,5-a]-pyrazin-2-carbonylamino]-2-[benzyloxycarbonylamino]-propionsäure

5. Verbindung gemäß Anspruch 1, 3-[2-(Guanidinocarbonylmethyl)-2,3-dihydro-3-oxo-[1,2,4]triazolo[4,3-a]pyridin-6-carbonylamino]-2-[(phenylmethoxy)carbonylamino]-propionsäure

6. Verbindung gemäß Ansprüch 1, 2-(Benzyloxycarbonylamino)-3-[[2-(2-(guanidylcarbonyl)-1-ethyl)-5-benzimidazolyl]-carbonylamino]propionsäure

7. Verbindung gemäß Anspruch 1, 3-[2-(2-Guanidinocarbonyl-1-ethyl)-2,3-dihydro-3-oxo-[1,2,4]triazolo[4,3-a]pyridin-6-carbonylamino]-2-[(phenylmethoxy)carbonylami no]propionsäure

8. Verbindung gemäß Anspruch 1, 3-[2-(3-Guanidinocarbonyl-1-propyl)-2,3-dihydro-3-oxo-[1,2,4]triazolo[4,3-a]pyridin-6-carbonylamino]-2-[(phenylmethoxy)carbonylam ino]propionsäure

9. Verbindung gemäß Ansprüch 1, 2-Benzyloxycarbonylamino-3-[6-(guanidinocarbonylmethyloxy)-3,4-dihydro-1-oxo-2(1H)-isochinolyl]propionsäure

10. Verfahren zur Herstellung einer Verbindung der Formel I gemäß einem oder mehreren der Ansprüche 1 bis 2, **dadurch gekennzeichnet, daß** man durch Fragmentkondensation zwei oder mehrere Fragmente, die sich retrosynthetisch aus der Formel I ableiten lassen, verknüpft.

11. Verbindung der Formel I gemäß einem oder mehreren der Ansprüche 1 bis 2 und/oder ihre physiologisch verträglichen Salze zur Verwendung als Arzneimittel.

12. Verbindung der Formel I gemäß einem oder mehreren der Ansprüche 1 bis 2 und/oder ihre physiologisch verträglichen Salze zur Verwendung als Inhibitor der Knochenresorption durch Osteoclasten, als Inhibitor von Tumorwachstum oder Tumormetasierung, als Entzündungshemmer, zur Behandlung oder Prophylaxe von cardiovaskulären Erkrankungen, zur Behandlung oder Prophylaxe von Nephropathien oder Retinopathien, oder als Vitronectinrezeptor-Antagonist zur Behandlung oder Prophylaxe von Krankheiten, die auf der Wechselwirkung zwischen Vitronectinrezeptoren und deren Liganden bei Zell-Zell- oder Zell-Matrix-Interaktionsprozessen beruhen.

13. Pharmazeutische Zubereitungen enthaltend mindestens eine Verbindung der Formel I gemäß einem oder mehreren der Ansprüche 1 bis 2 und/oder ihre physiologisch verträglichen Salze neben pharmazeutisch einwandfreien Träger- und Zusatzstoffen.

## Claims

1. Compound of the formula I,
A-B-D-E-F-G (I)
wherein:
A = A₁, where
A1 = R²R³N-C(=NR²)NR²C(O)-,
B is (C₁-C₄)-alkanediyl,
D is a direct bond, (C₁-C₄)-alkanediyl,
E is 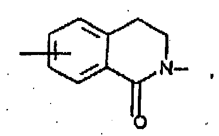
or 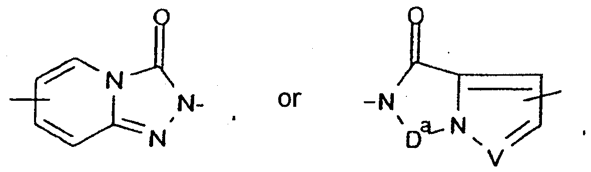 wherein V is N and D^{a} is CH₂-CH₂,
or 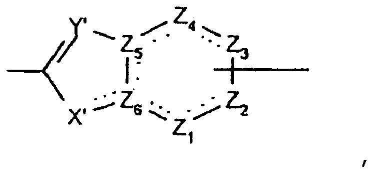 wherein:
X' is an -NR^{2b} group
R^{2b} being a hydrogen atom,
Y' is a nitrogen atom
Z₁, Z₂, Z₃ and Z₄, which may be the same or different, are methine groups, carbon atoms, wherein one of the radicals Z₁ to Z₄ must include a carbon atom and the other three of the radicals Z₁ to Z₄ are methine groups,
Z₅ and Z₆ are both carbon atoms,
F is a direct bond, (C₁-C₆)-alkanediyl, -CO-NR²-, each of which may be mono- or di-substituted with (C₁-C₄)-alkyl;
G is 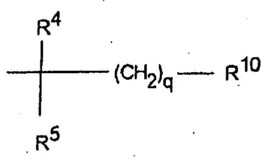
R², R³ are independently of one another H, (C₁-C₄)-alkyl,
R⁴ is R¹⁶OC(O)NH-, R¹⁶C(O)NH-
R⁵ is H, (C₁-C₆)-alkyl, (C₅-C₆) -cycloalkyl, (C₅-C₆)-cycloalkyl-(C₁-C₄)-alkyl, trifluoromethyl, pentafluoroethyl, phenyl, benzyl;
R⁸ is H, (C₁-C₄) -alkyl, (C₅-C₆) -cycloalkyl, (C₅-C₆)-cycloalkyl-(C₁-C₂)-alkyl, phenyl, benzyl, trifluoromethyl, pentafluoroethyl;
R¹⁰ is C(O)R¹¹;
R¹¹ is OH, (C₁-C₆) -alkoxy, phenoxy, benzyloxy, (C₁-C₄)-alkylcarbonyloxy-(C₁-C₄)-alkoxy, NH₂, mono- or di-(C₁-C₆-alkyl)-amino;
R¹⁶ is (C₁₀-C₁₄)-cycloalkyl, (C₁₀-C₁₄)-cycloalkyl-(C₁-C₄)-alkyl, which optionally may be mono- or di-substituted with (C₁-C₄)-alkyl, trifluoromethyl, phenyl, benzyl, (C₁-C₄)-alkoxy, phenoxy, benzyloxy, =O or mono- or di-(C₁-C₄-alkyl)-amino, wherein the cycloalkyl radicals are preferably 1-adamantyl or 2-adamantyl which may be substituted as described above;
n is 1 or 2; and
q is 0
in all of its stereoisomeric forms and mixtures thereof in any ratios, and its physiologically suitable salts.

2. Compound of the formula I according to Claim 1, in which the distance between R¹⁰ and the first nitrogen atom in A₁ along the shortest path between these atoms is from 12 to 13 covalent bonds, in all of its stereoisomeric forms and mixtures thereof in any ratios, and its physiologically suitable salts.

3. Compound according to Claim 1, (2S)-3-[5-(2-guanidinocarbonyl-ethyl)-4,5,6,7-tetrahydro-4-oxo-pyrazolo[1,5-a]-pyrazine-2-carbonylamino]-2-[benzyloxycarbonylamino]propanoic acid.

4. Compound according to Claim 1, (2S)-3-[5-(3-guanidinocarbonyl-propyl)-4,5,6,7-tetrahydro-4-oxo-pyrazolo[1,5-a]pyrazine-2-carbonylamino]-2-[benzyloxycarbonylamino]propanoic acid.

5. Compound according to Claim 1, 3-[2-(guanidinocarbonylmethyl)-2,3-dihydro-3-oxo[1,2,4]-triazolo[4,3-a]pyridine-6-carbonylamino]-2-[(phenylmethoxy)carbonylamino]propanoic acid.

6. Compound according to Claim 1, 2-benzyloxy-carbonylamino)-3-[[2-(2-(guanidylcarbonyl)-1-ethyl)-5-benzimidazolyl]-carbonylamino]propanoic acid.

7. Compound according to Claim 1, 3-[2-(2-guanidinocarbonyl-1-ethyl)-2,3-dihydro-3-oxo[1,2,4]-triazolo[4,3-a]pyridine-6-carbonylamino]-2-[(phenylmethoxy)carbonylamino]propanoic acid.

8. Compound according to Claim 1, 3-[2-(3-guanidinocarbonyl-1-propyl)-2,3-dihydro-3-oxo[1,2,4]-triazolo[4,3-a]pyridine-6-carbonylamino]-2-[(phenylmethoxy)carbonylamino]propanoic acid.

9. Compound according to Claim 1, 2-benzyloxycarbonylamino)-3-[6-guanidinocarbonylmethyloxy)-3,4-dihydro-1-oxo-2(1H)-isoquinolyl]propanoic acid.

10. Method of preparing a compound of the formula I according to either or both of Claims 1 and 2, **characterized in that** two or more fragments which can retro-synthetically be derived from the formula I are linked by fragment condensation.

11. Compound of the formula I according to either or both of Claims 1 and 2, and/or its physiologically suitable salts for use as medicament.

12. Compound of the formula I according to either or both of Claims 1 and 2, and/or its physiologically suitable salts for use as inhibitor of bone resorption by osteoclasts, as inhibitor of tumour growth or tumour metastasis, as anti-inflammatory agent, for treating or preventing cardiovascular diseases, for treating or preventing nephropathies or retinopathies, or as vitronectin receptor antagonist for treating or preventing diseases based on the interaction between vitronectin receptors and their ligands in cell-cell or cell-matrix interactive processes.

13. Pharmaceutical preparations, comprising at least one compound of the formula I according to either or both of Claims 1 and 2 and/or its physiologically suitable salts in addition to pharmaceutically acceptable carriers and additives.

## Revendications

1. Composé de formule (I)
A-B-D-E-F-G (I)
où :
A = A₁, avec
A₁ = R²R³N-C(=NR²)NR²C(O)-
B signifie (C₁-C₄)-alcanediyle ;
D signifie une liaison directe, (C₁-C₄)-alcanediyle,
E signifie 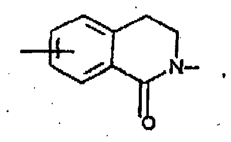
ou 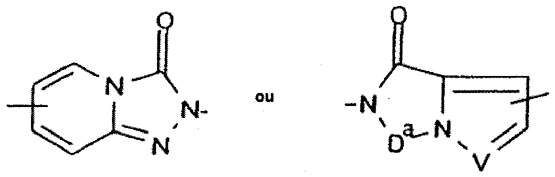 où V représente N et D^{a} représente CH₂-CH₂,
ou 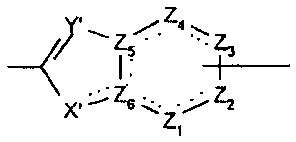 où
X' signifie un groupe -NR^{2b}
R^{2b} représentant un atome d'hydrogène,
Y' signifie un atome d'azote,
Z₁, Z₂, Z₃ et Z₄, qui peuvent être identiques ou différents, signifient des groupes méthine, des atomes de carbone, un des radicaux Z₁ à Z₄ devant contenir un atome de carbone et les trois autres des radicaux Z₁ à Z₄ étant des groupes méthine
Z₅ et Z₆ signifient à chaque fois un atome de carbone
F signifie une liaison directe, (C₁-C₆)-alcanediyle, -CO-NR²-, qui peuvent à chaque fois être monosubstitués ou disubstitués par (C₁-C₄)-alkyle ;
G signifie 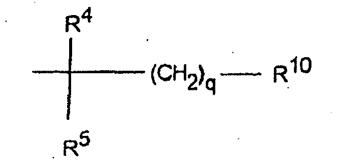
R², R³ signifient, indépendamment l'un de l'autre H, (C₁-C₄)-alkyle ;
R⁴ signifie R¹⁶OC(O)NH-, R¹⁶C(O)NH-
R⁵ signifie H, (C₁-C₆)-alkyle, (C₅-C₆)-cycloalkyle, (C₅-C₆)-cycloalkyl-(C₁-C₄)-alkyle, trifluorométhyle, pentafluoroéthyle, phényle, benzyle ;
R⁸ signifie H, (C₁-C₄)-alkyle, (C₅-C₆)-cycloalkyle, (C₅-C₆)-cycloalkyl-(C₁-C₂)-alkyle, phényle, benzyle, trifluorométhyle, pentafluoroéthyle ;
R¹⁰ signifie C(O)R¹¹ ;
R¹¹ signifie OH, (C₁-C₆)-alcoxy, phénoxy, benzyloxy, (C₁-C₄)-alkylcarbonyloxy-(C₁-C₄)-alcoxy, NH₂, mono-(C₁-C₆-alkyl)-amino ou di-(C₁-C₆-alkyl)-amino ;
R¹⁶ signifie (C₁₀-C₁₄) -cycloalkyle, (C₁₀-C₁₄) -cycloalkyl-(C₁-C₄)-alkyle, qui peuvent le cas échéant être monosubstitués ou disubstitués par (C₁-C₄)-alkyle, trifluorométhyle, phényle, benzyle, (C₁-C₄)-alcoxy, phénoxy, benzyloxy, =O ou mono-(C₁-C₄-alkyl)-amino ou di-(C₁-C₄-alkyl)-amino, les radicaux cycloalkyle étant de préférence 1-adamantyle ou 2-adamantyle, qui peuvent être substitués comme décrit ci-dessus ;
n vaut 1 ou 2 ; et
q vaut 0
dans toutes ses formes stéréo-isomères et leurs mélanges, dans tous les rapports, et ses sels physiologiquement acceptables.

2. Composé de formule I selon la revendication 1, dans lequel la distance entre R¹⁰ et le premier atome de N dans A₁ le long du chemin le plus court entre ces atomes comporte 12 à 13 liaisons covalentes, dans toutes ses formes stéréo-isomères et leurs mélanges, dans tous les rapports, et ses sels physiologiquement acceptables.

3. Composé selon la revendication 1, l'acide (2S)-3-[5-(2-guanidinocarbonyléthyl)-4,5,6,7-tétrahydro-4-oxopyrazolo[1,5-a]-pyrazine-2-carbonylamino]-2-[benzyloxycarbonylamino]-propionique.

4. Composé selon la revendication 1, l'acide (2S)-3-[5-(3-guanidinocarbonylpropyl)-4,5,6,7-tétrahydro-4-oxopyrazolo[1,5-a]-pyrazine-2-carbonylamino]-2-(benzyloxycarbonylamino]-propionique.

5. Composé selon la revendication 1, l'acide 3-[2-(guanidinocarbonylméthyl)-2,3-dihydro-3-oxo-[1,2,4]triazolo[4,3-a]pyridine-6-carbonylamino]-2-[(phénylméthoxy)carbonylamino]-propionique.

6. Composé selon la revendication 1, l'acide 2-(benzyloxycarbonylamino)-3-[[2-(2-(guanidylcarbonyl)-1-éthyl)-5-benzimidazolyl]-carbonylamino]propionique.

7. Composé selon la revendication 1, l'acide 3-[2-(2-guanidinocarbonyl-1-éthyl)-2,3-dihydro-3-oxo-[1,2,4]triazolo[4,3-a]pyridine-6-carbonylamino]-2-[(phénylméthoxy)carbonylamino]propionique.

8. Composé selon la revendication 1, l'acide 3-[2-(3-guanidinocarbonyl-1-propyl)-2,3-dihydro-3-oxo-[1,2,4]triazolo[4,3-a]pyridine-6-carbonylamino]-2-[(phénylméthoxy)carbonylamino]propionique.

9. Composé selon la revendication 1, l'acide 2-benzyloxycarbonylamino-3-[6-(guanidinocarbonylméthyloxy)-3,4-dihydro-1-oxo-2(1H)-isoquinoléyl]propionique.

10. Procédé pour la préparation d'un composé de formule I selon l'une ou plusieurs des revendications 1 à 2, **caractérisé en ce qu'**on lie, par condensation de fragments, deux fragments ou plus, qui peuvent être dérivés par rétrosynthèse de la formule I.

11. Composé de formule I selon l'une ou plusieurs des revendications 1 à 2 et/ou ses sels physiologiquement acceptables pour une utilisation comme médicament.

12. Composé de formule I selon l'une ou plusieurs des revendications 1 à 2 et/ou ses sels physiologiquement acceptables pour une utilisation comme inhibiteur de la résorption osseuse par des ostéoclastes, comme inhibiteur de la croissance tumorale ou de la formation de métastases tumorales, comme anti-inflammatoire, pour le traitement ou la prophylaxie de maladies cardiovasculaires, pour le traitement ou la prophylaxie de néphropathies ou de rétinopathies, comme antagoniste du récepteur de la vitronectine pour le traitement ou la prophylaxie de maladies qui reposent sur l'interaction entre les récepteurs de la vitronectine et leurs ligands dans des processus d'interaction cellule-cellule ou cellule-matrice.

13. Préparations pharmaceutiques, contenant au moins un composé de formule I selon l'une ou plusieurs des revendications 1 à 2 et/ou ses sels physiologiquement acceptables, en plus de supports et d'additifs pharmaceutiquement inoffensifs.
